# EUROPÄISCHE PATENTSCHRIFT

(11) **EP 2 996 596 B1**
(45) Veröffentlichungstag und Bekanntmachung des Hinweises auf die Patenterteilung: **24.04.2019**
(21) Anmeldenummer: 14724716.7
(22) Anmeldetag: 14.05.2014
(51) Int. Cl.: A61F 7/12, A61B 18/00, A61F 7/00, A61M 25/00, A61B 18/02, A61M 25/10, A61M 25/01

(54) **MEDIZINISCHER KATHETER ZUR HYPOTHERMISCHEN BEHANDLUNG, BEHANDLUNGSSYSTEM MIT EINEM DERARTIGEN KATHETER UND HERSTELLUNGSVERFAHREN**
MEDICAL CATHETER FOR HYPOTHERMIC TREATMENT, TREATMENT SYSTEM WITH SUCH A CATHETER, AND PRODUCTION METHOD
CATHÉTER DE TRAITEMENT MÉDICAL PAR HYPOTHERMIE, SYSTÈME DE TRAITEMENT ÉQUIPÉ D'UN TEL CATHÉTER ET PROCÉDÉ DE FABRICATION

(30) Priorität: 14.05.2013 DE 102013104948
(43) Veröffentlichungstag der Anmeldung: 23.03.2016
(73) Patentinhaber: Adceris GmbH & Co. KG, 75177 Pforzheim (DE)
(72) Erfinder: WOLFERTZ, Julia, 76131 Karlsruhe (DE); CATTANEO, Giorgio, 76199 Karlsruhe (DE); BÜCHERT, Michael, 75015 Bretten (DE)
(74) Vertreter: Kilchert, Jochen
(86) Internationale Anmeldenummer: PCT/EP2014/059837
(87) Internationale Veröffentlichungsnummer: WO 2014/184238

(56) Entgegenhaltungen:
- EP-A1- 0 729 766
- US-A- 4 563 180
- US-A1- 2012 041 419
- US-A1- 2012 143 131
- US-A1- 2012 150 107

## Beschreibung

Die Erfindung betrifft einen medizinischen Katheter zur hypothermischen Behandlung nach dem Oberbegriff des Patentanspruchs 1. Ferner betrifft die Erfindung ein Behandlungssystem mit einem derartigen Katheter und ein Herstellungsverfahren. Ein Katheter der eingangs genannten Art ist beispielsweise aus US 2002/0082556 A1 bekannt.

Die therapeutische Hypothermie wird genutzt, um insbesondere Blut im Bereich der Halsschlagader oder anderen zerebralen Blutgefäßen zu kühlen. Für den Einsatz der therapeutischen Hypothermie in zerebralen Blutgefäßen sind einige Herausforderungen zu bewältigen. Einerseits sind die Blutgefäße im zerebralen Bereich vergleichsweise klein und andererseits oft stark gewunden, so dass sich hohe Anforderungen an die Flexibilität des Katheters stellen. Gleichzeitig ist es für eine effiziente, lokale Kühlung zweckmäßig, wenn der Katheter eine isolierende Wirkung aufweist, um sicherzustellen, dass die Kühlung hauptsächlich an einem Punkt des Katheters, insbesondere an der Katheterspitze, erfolgt. Die Effizienz der katheterbasierenden Hypothermie erhöht sich außerdem mit dem Volumenstrom der Kühlflüssigkeit, die durch die Kanäle des Katheters geführt wird. Daher wird auch angestrebt, die Querschnittsdurchmesser der Kühlkanäle möglichst groß zu gestalten. Dennoch soll der Katheter einen kleinen Außendurchmesser und eine hohe Biegeflexibilität aufweisen, um auch periphere Blutgefäße gut erreichen zu können.

Zur Zuführung des Katheters wird üblicherweise eine Schleuse genutzt, die einen begrenzten Innendurchmesser aufweist. Bei bekannten Hypothermiekathetern, wie beispielsweise dem Katheter aus der eingangs genannten US 2002/0082556 A1, wird als Wärmetauscherelement ein Ballon eingesetzt. Der Ballon weist im komprimierten Zustand einen Außendurchmesser auf, der größer als der Katheterschlauchdurchmesser in einem proximalen Abschnitt des Katheterschlauchs ist. Somit wird der verbleibende Ringraum zwischen dem proximalen Katheterabschnitt und der Schleuse nicht genutzt.

Druckschrift US 2012/150107 A1 offenbart einen Ballonkatheter zur kryogenischen Ablation, wobei der Katheter zwei Ballone trägt, die ineinander angeordnet sind. Ein äußerer Ballon dient dabei als Sicherheitsballon, der bei einer möglichen Leckage eines inneren Ballons ein Austreten der kryogenischen Flüssigkeit verhindert. Der Katheter umfasst außerdem ein Inflationslumen, das einerseits einen inneren Katheterschlauch aufnimmt und andererseits kryogenische Flüssigkeit in den inneren Ballon leitet. Innerhalb des inneren Ballons entspannt die kryogenische Flüssigkeit und wird dadurch in einen gasförmigen Zustand überführt. Das dabei entstehende Gas kann über dasselbe Inflationslumen des Katheterschlauchs abgeführt werden.

Die Aufgabe der Erfindung besteht darin, einen medizinischen Katheter zur hypothermischen Behandlung anzugeben, der sich gut in kleine gewundene Gefäße vorschieben lässt und eine zweckmäßige Isolierwirkung aufweist. Ferner soll der in einer Schleuse zur Verfügung stehende Raum optimal nutzbar sein.

Eine weitere Aufgabe der Erfindung besteht darin, ein Behandlungssystem mit einem derartigen Katheter und ein Herstellungsverfahren anzugeben.

Erfindungsgemäß wird diese Aufgabe hinsichtlich des Katheters durch den Gegenstand des Patentanspruchs 1, hinsichtlich des Behandlungssystems durch den Gegenstand des Patentanspruchs 14 und hinsichtlich des Herstellungsverfahrens durch den Gegenstand des Patentanspruchs 15 gelöst.

So beruht die Erfindung auf dem Gedanken, einen medizinischen Katheter zur hypothermischen Behandlung mit einem Katheterschlauch anzugeben, der wenigstens einen Durchgangskanal und wenigstens zwei Temperierkanäle aufweist. Der Katheter weist wenigstens ein Wärmetauscherelement, insbesondere einen expandierbaren Ballon, auf, das in einem distalen Katheterabschnitt des Katheterschlauchs angeordnet ist. Das Wärmetauscherelement ist mit den Temperierkanälen fluidverbunden, so dass ein Temperierkreislauf gebildet ist. Erfindungsgemäß weist der Katheterschlauch im distalen Katheterabschnitt einen kleineren Außendurchmesser als in einem proximalen Katheterabschnitt auf.

Indem der Katheterschlauch im distalen Katheterabschnitt einen kleineren Außendurchmesser als im proximalen Katheterabschnitt aufweist, wird der in einer Schleuse zur Verfügung stehenden Raum gut genutzt. In dem verbleibenden Aufnahmeraum, zwischen dem Außendurchmesser des proximalen Katheterabschnitts und dem Außendurchmesser des distalen Katheterabschnitts kann das Wärmetauscherelement angeordnet werden, ohne den Außendurchmesser des Katheterschlauchs insgesamt zu erhöhen. So wird der in einer Schleuse zur Verfügung stehende Platz optimal ausgefüllt.

Grundsätzlich kann der Katheter auch ohne eine Schleuse eingesetzt werden. Insofern kann der Katheter selbst die Funktion einer Schleuse übernehmen bzw. als eine solche wirken. Diese Ausführungsform wird im Rahmen der vorliegenden Anmeldung als Schleusenvariante bezeichnet. Demnach kann der Katheter direkt durch die Gefäßwand in ein Blutgefäß eingeführt werden. Die erfindungsgemäße Konstruktion des Katheters hat auch in diesem Fall Vorteile. Insbesondere durch den im distalen Katheterabschnitt reduzierten Querschnittsdurchmesser, in dem auch das Wärmetauscherelement angeordnet ist, wird erreicht, dass der Gesamtdurchmesser des Katheters, d.h. des Katheterschlauchs einschließlich des Wärmetauscherelements, bei der Zufuhr durch die Gefäßwand kaum oder keine traumatischen Effekte zeigt. Bei der Schleusenvariante (der Katheter übernimmt zusätzlich die Funktion einer Schleuse) wird folglich eine Traumatisierung von Gewebe beim Einführen des Katheters reduziert.

Mit anderen Worten kann der Katheter bzw. der Katheterschlauch in besonderen Ausführungsformen der Erfindung selbst eine Schleuse bilden, durch welche wiederum ein Katheter, insbesondere ein Führungskatheter, zugeführt werden kann. Auch in diesem Fall ist eine distale Durchmesserreduzierung des Schafts vorgesehen, sodass nach Aufbringen wenigstens eines expandierbaren Wärmetauscherelements, insbesondere eines Ballons, der Gesamtaußendurchmesser im komprimierten Zustand des Wärmetauscherelements distal und proximal annähernd identisch ist.

Der distale Katheterabschnitt kann eine höhere Biegeflexibilität als der proximale Katheterabschnitt aufweisen. Auf diese Weise ist sichergestellt, dass der Katheterschlauch gut in stark gekrümmte Gefäße einschwenken kann. So können auch Behandlungsorte in stark gewundenen Gefäßen gut erreicht werden.

Bei einer besonders bevorzugten Ausführungsform der Erfindung ist vorgesehen, dass der Katheterschlauch im distalen Katheterabschnitt eine kleinere Wandstärke als im proximalen Katheterabschnitt aufweist. Mit anderen Worten ist die Wandstärke des proximalen Katheterabschnitts größer als die des distalen Katheterabschnitts. Damit wird erreicht, dass der proximale Katheterabschnitt eine erhöhte Steifigkeit aufweist. Der Katheter lässt sich so gut in ein Blutgefäß schieben, da die Vorschubkraft durch die erhöhte Wandstärke im proximalen Katheterabschnitt längsaxial an den distalen Katheterabschnitt übertragen wird. Ein Einknicken des proximalen Katheterabschnitts wird so vermieden. Gleichzeitig erhöht die Wandstärke im proximalen Katheterabschnitt die Isolierwirkung des Katheterschlauchs, so dass Temperierfluid mit geringem Temperaturverlust bis an den distalen Katheterabschnitt geleitet werden kann.

Vorzugsweise besteht ein lineares Verhältnis zwischen der Änderung der Wandstärke und der Änderung des Querschnittsdurchmessers. Der proximale Katheterabschnitt und der distale Katheterabschnitt weisen daher eine geometrische Ähnlichkeit auf. Mit anderen Worten ist die Querschnittsgeometrie des distalen Katheterabschnitts durch eine Skalierung der Querschnittsgeometrie des proximalen Katheterabschnitts gebildet. Konkret können alle Querschnittdimensionen (Außendurchmesser, Innenprofil und Wandstärke) in einem Verhältnis zueinander stehen, das im proximalen Katheterabschnitt und im distalen Katheterabschnitt identisch oder zumindest ähnlich ist.

In bevorzugten Ausführungsformen der Erfindung ist außerdem vorgesehen, dass der Katheterschlauch ein konstantes Querschnittsprofil, insbesondere einen konstanten Innendurchmesser, aufweist. Die Differenz zwischen dem Außendurchmesser des Katheterschlauchs im distalen Katheterabschnitt und im proximalen Katheterabschnitt ergibt sich vorzugsweise aus einer Reduzierung der Wandstärke, insbesondere ohne das innere Querschnittsprofil des Katheterschlauchs zu beeinflussen. Vielmehr kann sich das innere Querschnittsprofil unverändert über die gesamte Länge des Katheterschlauchs erstrecken. Vorzugsweise ist der Innendurchmesser des Katheterschlauchs über die gesamte Länge konstant.

Das Wärmetauscherelement, insbesondere der Ballon, des erfindungsgemäßen medizinischen Katheters kann derart komprimierbar sein, dass das Wärmetauscherelement im komprimierten Zustand einen Querschnittsdurchmesser aufweist, der höchstens dem Außendurchmesser des proximalen Katheterabschnitts entspricht. Konkret kann zwischen einer Außenumfangsebene des distalen Katheterabschnitts und einer Außenumfangsebene des proximalen Katheterabschnitts am distalen Katheterabschnitt ein Aufnahmeraum für das Wärmetauscherelement gebildet sein. Das Wärmetauscherelement ist dabei so komprimierbar, dass das Wärmetauscherelement vollständig innerhalb des Aufnahmeraums anordenbar ist. Ein derart angepasstes Wärmetauscherelement stellt sicher, dass der Katheterschlauch insgesamt einen maximalen Außendurchmesser aufweist, der durch den Außendurchmesser des proximalen Katheterabschnitts bestimmt ist. Im proximalen Katheterabschnitt weist der Katheterschlauch den größten Außendurchmesser auf, so dass der Raum innerhalb einer Schleuse im distalen Katheterabschnitt optimal genutzt werden kann. Vorgenanntes gilt insbesondere bei Verwendung des Katheters mit einer zusätzlichen Schleuse. Falls hingegen keine Schleuse zur Einfuhr des Katheters in ein Blutgefäß eingesetzt wird, sondern der Katheter selbst eine Schleuse bildet, wird durch die Begrenzung des Außendurchmessers die Traumatisierung von Gewebe während der Einfuhr des Katheters in das Blutgefäß reduziert.

Insbesondere kann das Wärmetauscherelement eine Wandstärke von höchstens 30 µm, insbesondere höchstens 25 µm, insbesondere höchstens 20 µm, insbesondere höchstens 15 µm, aufweisen. Mindestens beträgt die Wandstärke vorzugsweise 5 µm, insbesondere mindestens 10 µm. Der Querschnittsdurchmesser des Wärmetauscherelements beträgt vorzugsweise höchstens 5 mm, insbesondere höchstens 4,5 mm, insbesondere höchstens 4,0 mm, insbesondere höchstens 3,5 mm, insbesondere höchstens 3 mm im expandierten Zustand. Die Untergrenze für den Querschnittsdurchmesser des Wärmetauscherelements beträgt vorzugsweise 2,5 mm. Der Querschnittsdurchmesser des Wärmetauscherelements wird für die Anwendung des Katheters vorzugsweise so gewählt, dass er im expandierten Zustand höchstens 50% des Innenquerschnittsdurchmessers des Zielgefäßes beträgt. Insbesondere sollte die Querschnittsfläche des expandierten Wärmetauscherelements höchstens die Hälfte der Innenquerschnittsfläche des Blutgefäßes am Behandlungsort betragen. So bleibt während der Behandlung ein ausreichender Blutfluss um das Wärmetauscherelement erhalten.

Es ist grundsätzlich möglich, dass der Katheter mehrere Wärmetauscherelemente aufweist. Insbesondere können zwei, drei, vier, fünf oder sechs Wärmetauscherelemente, beispielsweise Ballone, am distalen Katheterabschnitt angeordnet sein. Die Länge des im expandierten Zustand zylindrischen Mittelteils der Wärmetauscherelemente bzw. Ballone beträgt vorzugweise zwischen 10 mm und 40 mm, insbesondere zwischen 15 mm und 30 mm, vorzugsweise 20 mm. Der zylindrische Mittelteil wird im expandierten Zustand von zwei längsaxialen Endabschnitten begrenzt, die kegelförmig ausgebildet sind. Dabei beträgt der Kegelwinkel (an der Kegelspitze) zwischen 20 Grad und 60 Grad, vorzugsweise 45 Grad. Besonders vorteilhaft ist es, wenn die Länge des gesamten distalen Katheterabschnitts, also vom ersten bis zum letzten Wärmetauscherelement bzw. Ballon, zwischen 40 mm und 120 mm, insbesondere zwischen 60 mm und 100 mm, vorzugsweise 80 mm, beträgt. Diese Länge gewährleistet, dass der in der gemeinsamen Halsschlagader (arteria carotis communis) zur Verfügung stehende Platz bestmöglich nutzbar ist.

Generell können mehrere Wärmetauscherelemente vorgesehen sein, die unterschiedliche Querschnittsdurchmesser aufweisen. Vorzugsweise reduziert sich der Querschnittsdurchmesser der Wärmetauscherelemente in längsaxialer Richtung des Katheters. Dabei können distal angeordnete Wärmetauscherelemente jeweils einen kleineren Querschnittsdurchmesser als proximal benachbarte Wärmetauscherelemente aufweisen. Besonders bevorzugt ist es, wenn wenigstens ein am weitersten distal angeordnetes Wärmetauscherelement einen Querschnittsdurchmesser aufweist, der derart klein ist, dass das Wärmetauscherelement im expandierten Zustand in die innere Halsschlagader (arteria carotis interna) einführbar ist. Es ist auch möglich, dass innerhalb des distalen Katheterabschnitts ein, zwei oder drei Wärmetauscherelemente, die in einem distalen Bereich des distalen Katheterabschnitts angeordnet sind, einen kleineren Querschnittsdurchmesser im expandierten Zustand aufweisen, als ein oder mehrere Wärmetauscherelemente, die in einem proximalen Bereich des distalen Katheterabschnitts angeordnet sind. Insbesondere können die Wärmetauscherelemente mit relativ kleinem Querschnittsdurchmesser, die vorzugsweise im distalen Bereich des distalen Katheterabschnitts angeordnet sind, in die innere Halsschlagader eingeführt werden , wogegen Wärmetauscherelemente mit relativ großem Querschnittsdurchmesser, die vorzugsweise im proximalen Bereich des distalen Katheterabschnitts angeordnet sind, in der gemeinsamen Halsschlagader verbleiben.

Der distale Bereich des distalen Katheterabschnitts kann eine Länge aufweisen, die zwischen 20 mm und 60 mm, vorzugsweise 40 mm, beträgt. Die Länge des proximalen Bereichs des distalen Katheterabschnitts kann zwischen 60 mm und 100 mm, insbesondere 80 mm, betragen. Die Wärmetauscherelemente im distalen Bereich des distalen Katheterabschnitts weisen im expandierten Zustand vorzugsweise einen Querschnittsdurchmesser auf, der zwischen 0,5 mm und 1,5 mm, insbesondere 1 mm, kleiner als im proximalen Bereich des distalen Katheterabschnitts ist.

Mit anderen Worten kann sich der Querschnittsdurchmesser in proximaler Richtung von Wärmetauscherelement zu Wärmetauscherelement erhöhen, wobei die Differenz des Querschnittdurchmessers benachbarter Wärmetauscherelemente vorzugsweise zwischen 0,5 mm und 1,5 mm beträgt. Die Differenz des Querschnittsdurchmessers zwischen dem am weitersten proximal und dem am weitersten distal angeordneten Wärmetauscherelement beträgt vorzugsweise zwischen 0,5 mm und 3 mm, insbesondere zwischen 0,5 mm und 2 mm, insbesondere zwischen 0,5 mm und 1 mm.

Wenn mehrere Wärmetauscherelemente im distalen Katheterabschnitt vorgesehen sind, können diese einen Abstand zueinander haben, der höchstens 8 mm, insbesondere höchstens 6 mm, insbesondere höchstens 4 mm, insbesondere höchstens 3 mm, beträgt. Vorzugsweise beträgt der Abstand zwischen zwei Wärmetauscherelementen wenigstens 1 mm, insbesondere wenigstens 2 mm. Der Abstand zwischen den Wärmetauscherelementen bzw. Ballonen wird im Sinne der vorliegenden Offenbarung zwischen den Kontaktstellen der Wärmetauscherelemente mit dem Katheterschlauch bzw. der Außenwand des Katheterschlauchs ermittelt.

Durch den begrenzten Abstand zwischen den Wärmetauscherelementen wird erreicht, dass einerseits der in einem Blutgefäß zur Verfügung stehende Platz gut genutzt und andererseits eine hohe Biegeflexibilität des distalen Katheterabschnitts aufrechterhalten wird. Die Wärmetauscherelemente bzw. Ballone können mit dem Katheterschlauch verklebt oder daran angeschweißt sein. Es ist auch möglich, die mehreren Ballone getrennt voneinander herzustellen oder einstückig auszubilden. Bei der einstückigen Ausbildung ist vorgesehen, dass der Ballon verjüngte bzw. verengte Bereich aufweist, die mit dem Katheter verbunden werden. Die Zuführung von Kühlflüssigkeit in die verschiedene Ballone kann parallel oder seriell erfolgen. Bei serieller Anordnung wird der entsprechende Temperierkanal auf Höhe jedes einzelnen Ballons verschlossen, damit die Kühlflüssigkeit, die aus einem Ballon austritt und wieder in den Kanal eintritt, dann in den nächsten Ballon umgeleitet wird, und keinen Shunt (Bypass) durch den Temperierkanal bildet. Der Verschluss kann beispielsweise durch einen Kleber realisiert werden.

Im Allgemeinen kann der Katheter Röntgenmarker aufweisen, die die Positionierung des Katheters unter Röntgenkontrolle erleichtern. Die Röntgenmarker können in Form von Coils oder röngtensichtbare Hülsen realisiert werden. Die Hülsen können einen Kunststoff, z.B. PU oder Pebax, umfassen, der mit Barium gemischt ist. Die Röntgenmarker können beispielsweise vor dem ersten (proximalen) Ballon und nach dem letzten (distalen) Ballon, d.h. im Bereich der Katheterspitze angeordnet sein. Es ist allerdings auch möglich, dass die Röntgenmarker zwischen einzelnen Wärmetauscherelementen bzw. Ballonen angeordnet sind. Insbesondere kann ein Röntgenmarker zwischen einem proximalen Bereich und einem distalen Bereich des distalen Katheterabschnitts zwischen zwei Ballonen positioniert sein. Der Röntgenmarker kann so die Ballone mit relativ kleinem Querschnittsdurchmesser von den Ballonen mit relativ großem Querschnittsdurchmesser optisch voneinander trennen. Die Röntgenmarker können den Katheter umschließen, oder innerhalb eines Kanals angeordnet werden. Eine Kombination (proximal um den Katheter, distal innerhalb eines Kanals) ist auch möglich. Der Coil ist vorzugsweise aus einem Platindraht oder einem Tantaldraht mit einem Durchmesser zwischen 40 und 250 µm, insbesondere zwischen 50 µm und 150 µm, vorzugsweise 100 µm, vorgesehen.

Der Katheterschlauch kann im Rahmen der vorliegenden Erfindung in einer ersten Variante einstückig ausgebildet sein. Insbesondere kann der Katheterschlauch einstückig durch einen Extrusionsprozess hergestellt sein. Durch die Änderung der Extrusionsparameter werden der Außendurchmesser des Katheterschlauchs und somit die Innendurchmesser des Durchgangskanals und der Temperierkanäle sowie die Wandstärken der Außenwand und der inneren Trennwände entlang des Katheterschlauchs variiert. Außerdem kann in einem einzigen Extrusionsvorgang das Material zur Herstellung des Katheterschlauchs geändert werden, so dass der distale Katheterabschnitt beispielsweise weicher bzw. flexibler als der proximale Katheterabschnitt ist. Insbesondere kann der distale Katheterabschnitt ein niedrigeres E-Modul als der proximale Katheterabschnitt aufweisen.

In einer zweiten Variante kann der Katheterschlauch mehrstückig, insbesondere mehrschichtig, aufgebaut sein.

Der einstückig ausgebildete Katheterschlauch kann beispielsweise durch ein Extrusionsverfahren hergestellt sein. Um den Außendurchmesser im distalen Katheterabschnitt zu reduzieren, kann durch ein mechanisches oder lasergesteuertes Abtragungsverfahren die Wandstärke des einstückig hergestellten Katheterschlauchs im distalen Katheterabschnitt reduziert werden. Alternativ kann vorgesehen sein, die verkleinerte Wandstärke bzw. den verkleinerten Außendurchmesser im distalen Katheterabschnitt bereits während des Extrusionsprozesses herzustellen.

Bei der mehrschichtigen Variante des Katheterschlauchs kann konkret vorgesehen sein, dass der Katheterschlauch zumindest im proximalen Katheterabschnitt eine mehrschichtige, insbesondere zweischichtige, Außenwand aufweist. Die Außenwand kann im proximalen Katheterabschnitt durch eine innere Schicht und eine äußere Schicht gebildet sein. Die Außenwand im distalen Katheterabschnitt kann durch die innere Schicht gebildet sein. Insbesondere kann die Außenwand im distalen Katheterabschnitt nur durch die innere Schicht gebildet sein. Die innere und die äußere Schicht sind vorzugsweise vollflächig miteinander verbunden. Beispielsweise kann die äußere Schicht aus einem Schrumpfschlauch, einem Coating oder einem weiteren Schlauch bestehen. Die äußere Katheterschicht kann durch ein Fügeverfahren, beispielsweise durch Lamination, mit der inneren Schicht verbunden sein. Grundsätzlich kann die Verbindung zwischen der äußeren Schicht und der inneren Schicht stoffschlüssig oder reibschlüssig erfolgen.

Die äußere und die innere Schicht können gleiche oder unterschiedliche Materialien aufweisen. Beispielsweise ist es möglich, dass die äußere Schicht und die innere Schicht gemeinsam durch einen Koextrusionsprozess hergestellt sind, wobei die äußere Schicht und die innere Schicht unterschiedliche Materialien aufweisen.

Durch die unterschiedlichen Schichten, insbesondere mit unterschiedlichen Materialien, können die Eigenschaften des proximalen Katheterabschnitts einfach variiert werden. Beispielsweise kann die äußere Schicht ein relativ hartes Material umfassen, so dass der proximale Katheterabschnitt durch die äußere Schicht verstärkt wird. Ferner kann für die äußere Schicht ein Material gewählt werden, das eine kleine Wärmeleitfähigkeit aufweist, um die Isolierung des proximalen Katheterabschnitts zu verbessern.

Bei einer bevorzugten Ausführungsform ist vorgesehen, dass die innere und/oder die äußere Schicht eine poröse, insbesondere schwammartige, Struktur und/oder Hohlkugeln umfasst. Insbesondere kann die äußere Schicht eine poröse bzw. schwammartige Struktur aufweisen. Durch die Porosität der inneren und/oder äußeren Schicht wird die Wärmeleitfähigkeit der jeweiligen Schicht reduziert. Auf diese Weise können Temperierkanäle gut gegenüber der Umgebung, beispielsweise umschwimmendem Blut, thermisch isoliert werden. Ein unkontrollierter Wärmeübergang vom Temperierfluid zur Umgebung, insbesondere im proximalen Katheterabschnitt, wird somit vermieden.

Zumindest die äußere Schicht kann eine Wärmeleitfähigkeit aufweisen, die weniger als 0,2 Wm⁻¹K⁻¹, insbesondere weniger als 0,15 Wm⁻¹K⁻¹, insbesondere weniger als 0,1 Wm⁻¹K⁻¹, insbesondere weniger als 0,08 Wm⁻¹K⁻¹, beträgt. Auf diese Weise wird eine verbesserte thermische Isolierung im proximalen Katheterabschnitt erreicht, so dass die Wärmeenergie des Temperierfluids hauptsächlich im Bereich des distalen Katheterabschnitts freigesetzt wird.

In einer weiteren bevorzugten Ausführungsform kann der Katheterschlauch einen Außenschlauch und einen Innenschlauch aufweisen. Der Außenschlauch kann größer als der Innenschlauch sein, so dass zwischen dem Außenschlauch und dem Innenschlauch ein Isolierraum gebildet ist. Im Allgemeinen kann zwischen dem Außenschlauch bzw. der äußeren Schicht und dem Innenschlauch bzw. der inneren Schicht ein Isolierraum freigehalten sein. Der Isolierraum kann durch einen Spalt bzw. Abstand zwischen der äußeren Schicht und der inneren Schicht gebildet sein. Damit wird die Isolierwirkung des Katheterschlauchs im proximalen Katheterabschnitt verbessert.

Vorzugsweise sind der Außenschlauch und der Innenschlauch exzentrisch zueinander ausgerichtet. Der Spalt bzw. der Isolierraum erstreckt sich dabei zweckmäßigerweise zumindest im Bereich der Temperierkanäle, um eine Isolierwirkung zumindest in diesem Bereich des Katheterschlauchs bereitzustellen. Der Spalt kann im Bereich der Temperierkanäle die größte Breite aufweisen.

Stellenweise können der Außenschlauch und der Innenschlauch miteinander verbunden sein. Konkret kann eine Verbindungslinie vorgesehen sein, die sich längsaxial entlang des Katheterschlauchs erstreckt und entlang welcher der Innenschlauch und der Außenschlauch miteinander verbunden, insbesondere verklebt oder verschweißt, sind. Vorzugsweise sind der Innenschlauch und der Außenschlauch im Bereich des Durchgangskanals miteinander verbunden, wobei der Durchgangskanal sich exzentrisch durch den Katheterschlauch erstreckt.

Zwischen dem Außenschlauch und dem Innenschlauch kann zumindest im Bereich der Temperierkanäle wenigstens ein Abstandshalter angeordnet sein. Auf diese Weise wird zwischen dem Außenschlauch und dem Innenschlauch ein Luftvolumen bereitgestellt, das zu einer verbesserten thermischen Isolierung führt.

Der Abstandshalter kann durch ein umgeformtes axiales Endes des Außenschlauchs gebildet sein. Insbesondere kann der Außenschlauch an einem distalen Ende umgeformt, beispielsweise gecrimpt, und mit dem Innenschlauch fluiddicht verbunden sein. Das umgeformte Ende kann gleichzeitig als Abstandshalter dienen. Grundsätzlich können mehrere Abstandshalter vorgesehen sein. Insbesondere können beide axialen Enden des Außenschlauchs einen Abstandshalter in Form einer Crimpung bilden. Alternativ oder zusätzlich können zwischen den axialen Enden des Außenschlauchs Abstandshalter angeordnet sein. Die Abstandshalter können einstückig mit dem Außenschlauch und/oder dem Innenschlauch ausgebildet sein. Es ist auch möglich, dass wenigstens ein Abstandshalter vorgesehen ist, der ein separates Bauteil bildet. Beispielsweise kann ein Ring zwischen dem Außenschlauch und dem Innenschlauch angeordnet sein, der einen Abstand zwischen dem Außenschlauch und dem Innenschlauch sicherstellt.

Bei einer weiteren bevorzugten Ausführungsform der vorliegenden Erfindung können zumindest die Temperierkanäle im proximalen Katheterabschnitt eine größere Querschnittsfläche als im distalen Katheterabschnitt aufweisen. Insbesondere kann der Katheterschlauch insgesamt einen Innendurchmesser aufweisen, der im proximalen Katheterabschnitt größer als im distalen Katheterabschnitt ist. Vorzugsweise ist die Wandstärke des Katheterschlauchs in dieser Variante konstant. Es ist allerdings auch möglich, dass sich entlang des Katheterschlauchs sowohl die Querschnittsfläche der Temperierkanäle und/oder des Durchgangskanals, als auch die Wandstärke ändert. Im Hinblick auf eine einfache und kostengünstige Herstellung ist es insbesondere bevorzugt, wenn sich die Dimensionen des distalen Katheterabschnitts insgesamt direkt proportional zu den Dimensionen des proximalen Katheterabschnitts verhalten. So kann der distale Katheterabschnitt eine Skalierung des proximalen Katheterabschnitts bilden.

Indem die Temperierkanäle im proximalen Katheterabschnitt eine größere Querschnittsfläche als im distalen Katheterabschnitt aufweisen, ist es möglich, einen relativ hohen Volumenstrom an Temperierfluid durch den Katheterschlauch zu pumpen, da durch die relativ große Querschnittsfläche im proximalen Katheterabschnitt geringe Druckverluste entstehen. Die Wärmetauscherfunktion des erfindungsgemäßen Katheters wird somit verbessert.

Der Katheterschlauch kann außerdem an einem distalen Axialende eine Katheterspitze tragen, die einen Querschnittsdurchmesser aufweist, der dem Querschnittsdurchmesser des distalen Katheterabschnitts entspricht oder größer ist. Die Katheterspitze kann gleichzeitig einen Verschluss für die Temperierkanäle bilden. Konkret kann die Katheterspitze die Temperierkanäle fluiddicht verschließen. Die Katheterspitze kann außerdem eine Durchgangsöffnung aufweisen, die mit dem Durchgangskanal fluchtet. Zwischen der Katheterspitze und dem proximalen Katheterabschnitt, also im distalen Katheterabschnitt, kann ein Wärmetauscherelement, insbesondere in Form eines Compliant-Ballons, angeordnet sein. Die Verwendung einer Katheterspitze der zuvor beschriebenen Art hat den Vorteil, dass beim Einführen des Katheterschlauchs in ein Blutgefäß das Wärmetauscherelement, insbesondere der Ballon, kaum Kontakt zur Katheterschleuse bzw. zur Gefäßwand aufweist. Eine reibhemmende Beschichtung des Ballons, wie sie bei bekannten Ballonkathetern üblich ist, ist somit nicht erforderlich. Durch den Verzicht auf eine zusätzliche, reibhemmende Beschichtung wird die Wärmeübertragung am Wärmetauscherelement verbessert, da kein zusätzlicher Wärmeleitwiderstand durch die reibhemmende Beschichtung vorhanden ist.

Die Katheterspitze kann geformt, beispielsweise abgerundet, sein. Die zwei Temperierkanäle können durch Kleben oder Schmelzen verschlossen werden.

Um den Raum innerhalb des Katheterschlauchs gut zu nutzen, hat es sich als vorteilhaft erwiesen, die Temperierkanäle derart zu gestalten, dass diese ein lungenflügelartiges Querschnittsprofil aufweisen. Konkret kann jeder Temperierkanal ein lungenflügelartiges Querschnittsprofil aufweisen. Die Querschnittsprofile der Temperierkanäle können zueinander spiegelbildlich ausgebildet sein. In dieser Konstellation ist der Durchgangskanal vorzugsweise exzentrisch bzw. seitlich im Katheterschlauch angeordnet.

Die einzelnen Kanäle des Katheterschlauchs, insbesondere der Durchgangskanal und die Temperierkanäle, können durch wenigstens eine Innenwand voneinander getrennt sein. Vorzugsweise ist die Innenwand stabil bzw. steif ausgebildet, so dass die Querschnittsflächen der Temperaturkanäle im Wesentlichen konstant bleiben, insbesondere sich nicht durch den Einfluss von Kräfte oder Druck ändern. Die Innenwand ist vorzugsweise flexibel, so dass die Querschnittsflächen der einzelnen Kanäle variabel sind. Insbesondere können die Temperierkanäle beim Durchströmen mit einem Temperierfluid aufgeweitet werden, insbesondere durch den Fluiddruck. Gleichzeitig kann die Querschnittsfläche des Durchgangskanals schrumpfen. Auf diese Weise kann der Katheterschlauch weiter miniaturisiert werden.

In diesem Zusammenhang wird darauf hingewiesen, dass der Katheter grundsätzlich mehrere Kanäle aufweisen kann. So können zusätzlich zu den Temperierkanälen oder dem Durchgangskanal weitere Kanäle beispielsweise für das Füllen eines Okklusionballons, für die Leitung von Sensorkabeln (Temperatur, Druck, Partialdruck, CO₂ oder O₂, Durchfluss, ...), oder Verabreichung von Medikamenten für die Lyse oder zur Neuroprotektion sowie zur Verabreichung von Kontrastmitteln oder Spasmolitika. Die Verabreichung kann alternativ über den Durchgangskanal erfolgen.

Im distalen Katheterabschnitt kann ein Aufnahmebereich für ein medizinisches Instrument vorgesehen sein, der einen größeren Querschnittsdurchmesser als der Durchgangskanal aufweist. So kann beispielsweise in expandiertes Rekanalisationselement, das durch den Durchgangskanal an einen Behandlungsort geführt wurde, in den Katheterschlauch zurückgezogen werden.

In der Variante der Erfindung bei welcher der Katheter zur Zufuhr in ein Blutgefäß über eine Schleuse (Kathetervariante) ausgebildet ist, kann im distalen Katheterabschnitt ein Aufnahmebereich für ein Konkrement bzw. ein Thrombus vorgesehen sein. Der Aufnahmebereich ermöglicht eine Entfernung des Konkrements bzw. des Thrombus nach einer Rekanalisation.

In der alternativen Variante der Erfindung, bei der der Katheter selbst als Schleuse wirkt und insofern direkt in das Blutgefäß eingeführt wird (Schleusenvariante) kann der distalen Katheterabschnitt ohne Aufnahmebereich ausgebildet sein. Die Handhabung der Schleusenvariante wird wie folgt durchgeführt: Zunächst wird ein Blutgefäß punktiert, meist mit einer Nadel, um einen Zugang zum Blutgefäß zu schaffen. Anschließend wird ein Führungsdraht in das Blutgefäß eingeführt und die Nadel wieder entfernt. Über den Führungsdraht wird nun der Katheterschlauch des Katheters geführt. Nachdem der Führungsdraht nun entfernt wurde, wird ein Führungskatheter, bevorzugt in der Größe 5 French oder 6 French, durch den Durchgangskanal des Katheterschlauchs vorgeschoben. Das eigentliche Rekanalisationselement, beispielsweise ein Fangkorb oder ein stentartiges System, wird durch einen Mikrokatheter, beispielsweise in der Größe 2 French, 2,5 French oder 3 French, innerhalb des Führungskatheters an den Behandlungsort geführt. Zur Stabilisierung des Mikrokatheters und zur Sondierung des Behandlungsorts kann ferner ein Führungsdraht eingesetzt werden. Der Thrombus kann nach dem Rekanalisationsvorgang in den Führungskatheter aspiriert und zusammen mit dem Führungskatheter durch den Durchgangskanal zurückgezogen werden. Alternativ oder zusätzlich kann die Aspiration bzw. die Entfernung des Thrombus auch durch den Durchgangskanal des Katheterschlauchs erfolgen. Der Thrombus kann somit vollständig entfernt werden, während der Kühlvorgang fortgeführt wird. Es ist auch möglich, den Mikrokatheter direkt durch den Durchgangskanal zu schieben und den Thrombus direkt in den Durchgangskanal zurückzuziehen, bedarfsweise unter Aspiration. Außerdem ist es denkbar, den Thrombus ohne Verwendung eines Rekanalisationselements und/oder Mikrokatheters direkt in den Führungskatheter oder den Durchgangskanal des Katheterschlauchs zu aspirieren.

Bei der Kathetervariante kann im proximalen Katheterabschnitt eine Markierung vorgesehen sein, die dem Anwender einen Hinweis darauf gibt, wenn das Wärmetauscherelement die Spitze der Schleuse erreicht oder bereits teilweise oder vollständig aus der Schleuse entlassen ist. Mit anderen Worten kann der Abstand von der Katheterspitze bis zur Markierung im proximalen Katheterabschnitt wenigstens der Länge der zu verwendenden Schleuse entsprechen.

In einer besonders bevorzugten Ausführungsform behält der Durchgangskanal einen konstanten Durchmesser bei. Insbesondere kann der Durchgangskanal im proximalen Katheterabschnitt und im distalen Katheterabschnitt denselben, konstanten Querschnittsdurchmesser aufweisen. Wegen des Aufnahmebereichs kann zur Zufuhr des Katheterschlauchs ein flexibler Innenkern, auch Dilatator genannt, eingesetzt werden, ohne dass ein traumatischer Spalt entsteht. Vielmehr reduziert sich die Querschnittsfläche der Transportkanäle in Richtung zur Katheterspitze so, dass an der Katheterspitze nur der Durchgangskanal bleibt, d.h. die Temperierkanäle enden vor der Katheterspitze. Der Katheter weist folglich ein besonderes atraumatisches Verhalten auf.

Gemäß einem nebengeordneten Aspekt beruht die Erfindung auf dem Gedanken ein Behandlungssystem mit einem zuvor erläuterten Katheter und einer selbstexpandierbaren Vorrichtung, insbesondere einem Rekanalisationselement, anzugeben, die mit einem länglichen Führungselement verbunden und mit dem Führungselement längsverschiebbar im Durchgangskanal angeordnet ist. In dieser Kombination kann der Katheter beispielsweise zur Kühlung von Blut eingesetzt werden, während gleichzeitig ein Thrombus aus dem Blutgefäß entfernt wird. Das Führungselement kann durch einen Transportdraht gebildet sein.

Das Rekanalisationselement ist bei der Kathetervariante vorzugsweise über einen Mikrokatheter an den Behandlungsort führbar, wobei der Mikrokatheter durch einen im Durchgangskanal des Katheterschlauchs angeordneten Führungskatheter an den Behandlungsort schiebbar ist. Mittels des Transportdrahts, der durch den Mikrokatheter geführt werden kann, kann das Rekanalisationselement an den Behandlungsort gebracht werden. Das Rekanalisationselement kann einen Aufnahmebereich für einen Thrombus bilden. Bei der Schleusenvariante kann ein Aspirationskatheter, beispielsweise in einer Größe von 5 French oder 6 French, durch den Durchgangskanal des Katheterschlauchs geführt und so über den Durchgangskanal ein Thrombus entfernt werden.

Ferner wird im Rahmen der vorliegenden Anmeldung ein Behandlungssystem offenbart und beansprucht, das einen zuvor beschriebenen Katheter und einen Führungskatheter aufweist, der längsverschieblich innerhalb des Durchgangskanals des Katheterschlauchs angeordnet oder positionierbar ist. In dieser Konstellation kann der Katheter vorzugsweise außerdem die Funktion einer Schleuse übernehmen, die somit vermeidbar ist.

Ein weiterer Aspekt der Erfindung betrifft ein Verfahren zur Herstellung des zuvor beschriebenen Katheters und/oder Behandlungssystems, wobei ein Innenschlauch innerhalb eines Außenschlauchs angeordnet wird derart, dass ein Endabschnitt des Innenschlauchs distal aus dem Außenschlauch vorragt, und wobei zumindest ein distales Ende des Außenschlauchs mit dem Innenschlauch, insbesondere durch Schrumpfen, Kleben oder Schweißen, fluiddicht verbunden wird. Das erfindungsgemäße Verfahren vereinfacht die Herstellung und ermöglicht eine schnelle und einfache Anpassung des Katheters. Beispielsweise können Außenschläuche unterschiedlicher Länge mit unterschiedlich langen Innenschläuchen kombiniert werden. So kann im Produktionsprozess schnell die Länge des distalen Katheterabschnitts geändert werden.

Es ist grundsätzlich möglich und im Rahmen der vorliegenden Anmeldung auch beansprucht, dass ein Verfahren zur Herstellung des zuvor beschriebenen Katheters und/oder Behandlungssystem einen Extrusionsprozess umfasst, wobei der Katheterschlauch einstückig aus einem einzigen Material oder unterschiedlichen Materialien extrudiert wird.

Die Erfindung wird im Folgenden anhand von Ausführungsbeispielen unter Bezugnahme auf die beigefügten, schematischen Zeichnungen näher erläutert. Darin zeigen
- Fig. 1: einen Längsschnitt durch den Katheterschlauch eines erfindungsgemäßen Katheters nach einem bevorzugten Ausführungsbeispiel;
- Fig. 2: einen Querschnitt durch den Katheterschlauch gemäß Fig. 1 im proximalen Katheterabschnitt;
- Fig. 3: einen Querschnitt durch den Katheterschlauch gemäß Fig. 2 im distalen Katheterabschnitt;
- Fig. 4: eine schematische Seitenansicht des Katheterschlauchs gemäß Fig. 1;
- Fig. 5: einen Querschnitt durch einen proximalen Katheterabschnitt eines einstückigen Katheterschlauchs;
- Fig. 6: einen Querschnitt durch den proximalen Katheterabschnitt eines mehrschichtigen Katheterschlauchs;
- Fig. 7: einen Querschnitt durch den proximalen Katheterabschnitt eines mehrschichtigen Katheterschlauchs, wobei Abstandshalter zur Bildung eines Isolierraums vorgesehen sind;
- Fig. 8: ein Querschnitt durch einen proximalen Katheterabschnitt eines Katheterschlauchs, wobei zwischen einem Außenschlauch und einem Innenschlauch ein ringförmiger, durchgehender Isolierraum vorgesehen ist;
- Fig. 9: eine Längsschnittansicht durch den Katheterschlauch gemäß Fig. 8;
- Fig. 10: eine Querschnittsansicht durch den proximalen Katheterabschnitt eines erfindungsgemäßen medizinischen Katheters nach einem weiteren bevorzugten Ausführungsbeispiel, wobei der Katheterschlauch einen Innenschlauch und einen exzentrisch angeordneten Außenschlauch aufweist;
- Fig. 11: eine Querschnittsansicht durch einen weiteren Katheterschlauch mit einem Innenschlauch und einem Außenschlauch, wobei zwischen dem Innenschlauch und dem Außenschlauch Abstandshalter angeordnet sind;
- Fig. 12: eine Querschnittsansicht durch den distalen Katheterabschnitt eines Katheterschlauchs gemäß den Fig. 5 bis 11;
- Fig. 13: eine Längsschnittansicht durch einen Katheterschlauch des erfindungsgemäßen Katheters nach einem weiteren bevorzugten Ausführungsbeispiel, wobei die Temperiervolumen im proximalen Katheterabschnitt eine größere Querschnittsfläche als im distalen Katheterabschnitt aufweisen;
- Fig. 14: eine Längsschnittansicht durch einen erfindungsgemäßen Katheter nach einem weiteren bevorzugten Ausführungsbeispiel mit einer am distalen Axialende angeordneten Katheterspitze, wobei zwischen der Katheterspitze und dem proximalen Katheterabschnitt ein Ballon als Wärmetauscherelement im komprimierten Zustand angeordnet ist;
- Fig. 15: die Längsschnittansicht gemäß Fig. 14, wobei der Ballon im expandierten Zustand dargestellt ist;
- Fig. 16: eine Seitenansicht eines Katheterschlauchs des erfindungsgemäßen Katheters nach einem bevorzugten Ausführungsbeispiel;
- Fig. 16a, b: jeweils eine Querschnittsansicht durch den Katheterschlauch gemäß Fig. 16;
- Fig. 17: eine Längsschnittansicht durch einen Katheterschlauch eines erfindungsgemäßen Katheters nach einem weiteren bevorzugten Ausführungsbeispiel mit einem im Durchgangskanal angeordneten Dilatator;
- Fig. 17a: eine Querschnittsansicht des Katheterschlauchs gemäß Fig. 17 mit dargestellter Schnittlinie;
- Fig. 17b,c: jeweils eine Querschnittsansicht durch den Katheterschlauch gemäß Fig. 17;
- Fig. 17d: eine Querschnittsansicht durch den Dilatator gemäß Fig. 17;
- Fig. 18: eine Längsschnittansicht durch einen Katheterschlauch des Katheters nach Fig. 17;
- Fig. 18a: eine Querschnittsansicht durch den Katheterschlauch gemäß Fig. 18 mit eingezeichneter Schnittlinie;
- Fig. 19: eine weitere Längsschnittansicht durch den Katheterschlauch gemäß Fig. 18;
- Fig. 19a: eine weitere Querschnittsansicht durch den Katheterschlauch gemäß Fig. 19 mit eingezeichneter Schnittlinie;

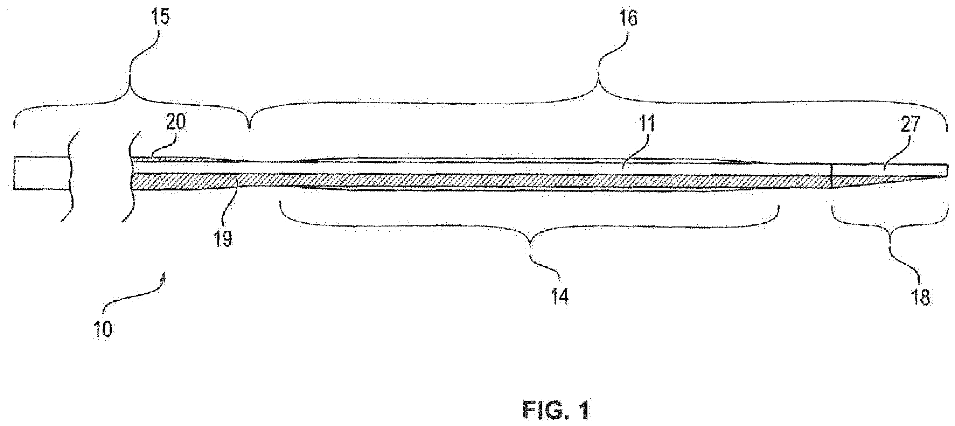

Fig. 1 zeigt einen Katheterschlauch 10 eines erfindungsgemäßen Katheters, wobei der Katheterschlauch 10 einen proximalen Katheterabschnitt 15 und einen distalen Katheterabschnitt 16 aufweist. Der distale Katheterabschnitt 16 umfasst eine Katheterspitze 18. Im distalen Katheterabschnitt 16 ist proximal zur Katheterspitze 18 ein Wärmetauscherelement 14 angeordnet. Das Wärmetauscherelement 14 ist vorzugsweise als expandierbarer Ballon ausgebildet.

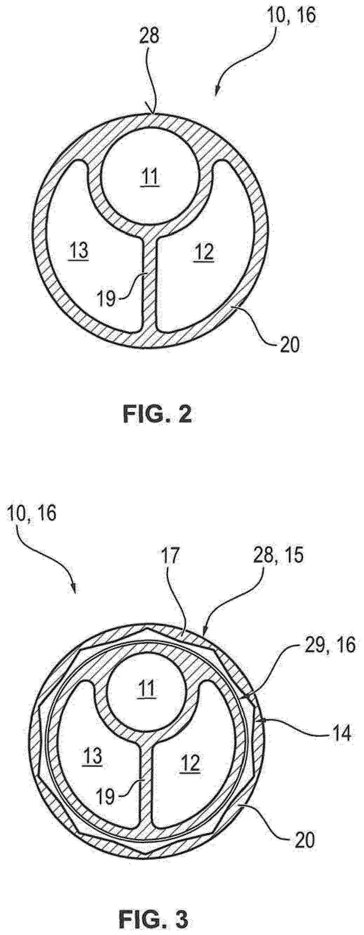

In Fig. 2 ist ein Querschnitt durch den Katheterschlauch 10 gemäß Fig. 1 im Übergangsbereich zwischen der Katheterspitze 18 und dem das Wärmetauscherelement 14 tragenden Teil des distalen Katheterabschnitts 16 gezeigt. Grundsätzlich weist der Katheterschlauch im distalen Katheterabschnitt 16, insbesondere auch im Bereich des Wärmetauscherelements 14, einen Querschnitt gemäß Fig. 2 auf.

Der Katheterschlauch 10 weist insgesamt eine Außenwand 20 auf, die den Katheterschlauch 10 nach außen begrenzt. Die Außenwand 20 bildet gleichzeitig eine Begrenzung für drei Kanäle 11, 12, 13, die sich längsaxial durch den Katheterschlauch 10 erstrecken. Insbesondere weist der Katheterschlauch 10 einen Durchgangskanal 11 auf, der sich vollständig durch den Katheterschlauch 10 erstreckt. Der Durchgangskanal 11 weist einen kreisförmigen Querschnitt auf. Vorzugsweise dient der Durchgangskanal 11 als Instrumentenkanal, d.h. für die Zuführung von Instrumenten durch den Katheterschlauch 10 an einen Behandlungsort. Derartige Instrumente können beispielsweise eine Rekanalisationsvorrichtung bzw. allgemein ein selbstexpandierbares Element umfassen. Der Durchgangskanal 11 ist bezüglich der Längsachse des Katheterschlauchs 10 exzentrisch angeordnet.

Ferner weist der Katheterschlauch 10 zwei Temperierkanäle 12, 13 auf. Die Temperierkanäle sind einerseits durch die Außenwand 20 begrenzt und andererseits durch eine Innenwand 19 voneinander und vom Durchgangskanal 11 getrennt. Die Innenwand 19 erstreckt sich durch den Katheterschlauch und weist vorzugsweise einen Y-förmigen Querschnitt auf. Die Temperierkanäle 12, 13 weisen jeweils einen lungenflügelartigen Querschnitt auf.

Der Längsschnitt gemäß Fig. 1 verläuft durch den Durchgangskanal 11 und die Innenwand 19, so dass in Fig. 1 die Temperierkanäle nicht erkennbar sind. Deutlich erkennbar ist in Fig. 1 hingegen der Ballon, der das Wärmetauscherelement 14 bildet. Das Wärmetauscherelement 14 ist komprimiert bzw. zusammengefaltet und erhöht somit im distalen Katheterabschnitt 16 die Außendimensionen des Katheterschlauchs 10 bereichsweise. Außerdem ist in Fig. 1 gut erkennbar, dass der Katheterschlauch 10 im distalen Katheterabschnitt 16 einen kleineren Querschnittsdurchmesser als im proximalen Katheterabschnitt 15 aufweist. Auf diese Weise ist in radialer Richtung bezogen auf den Katheterschlauch 10 im distalen Katheterabschnitt 16 ausreichend Platz außerhalb des Katheterschlauchs geschaffen, um den komprimierten Ballon bzw. das komprimierte Wärmetauscherelement 14 aufzunehmen. Konkret weist das Wärmetauscherelement 14 im komprimierten bzw. gefalteten Zustand insgesamt Außendimensionen auf, die nicht größer als der Querschnittsdurchmesser bzw. die Außendimensionen des Katheterschlauchs 10 im proximalen Katheterabschnitt 15 ist. Das Wärmetauscherelement 14 kann also im komprimierten bzw. gefalteten Zustand eine Außendimension aufweisen, die kleiner oder höchstens genauso groß wie die Außendimension des proximalen Katheterabschnitts 15 ist.

Im Allgemeinen kann vorgesehen sein, dass die Innenwand 19 flexibel ist. Insbesondere können mehrere flexible Innenwände 19 innerhalb des Katheterschlauchs 10 angeordnet sein, die die einzelnen Kanäle 11, 12, 13 voneinander trennen. Durch die Flexibilität der Innenwände können sich die Temperierkanäle 12, 13 ausdehnen, um einen höheren Volumenstrom eines Temperierfluids zu erlauben. Zur Durchführung eines Instruments durch den Durchgangskanal 11 können die Temperierkanäle 12, 13, beispielsweise durch Erzeugung eines Unterdrucks, komprimiert werden, so dass der Durchgangskanal 11 entsprechend aufgeweitet wird. Bevorzugt ist es, wenn die Innenwände des Kathetherschlauchs 10 derart stabil sind, dass eine Verformung des Durchgangskanals 11 und/oder der Temperierkanäle 12, 13 vermieden wird. Dennoch weist der Katheterschlauch 10 vorzugsweise eine hohe Biegeflexibilität auf.

Alternativ oder zusätzlich kann im distalen Katheterabschnitt 16 ein Aufnahmebereich vorgesehen sein, der zur Aufnahme eines medizinischen Instruments, insbesondere eines expandierten Instruments, angepasst ist. Der Aufnahmebereich weist vorzugweise eine Querschnittsfläche auf, die größer als die Querschnittsfläche des Durchgangskanals 11 ist. Insbesondere kann die Innenwand 19 in einem Abstand zum distalen Ende des Katheterschlauchs 10 enden, so dass der Katheterschlauch 10 im Aufnahmebereich im Wesentlichen einbautenfrei ist.

Ferner ist in Fig. 1 erkennbar, dass die Wandstärke der Außenwand 20 im proximalen Katheterabschnitt 15 größer als im distalen Katheterabschnitt 16 ist. Die Außenwand 20 dient gleichzeitig als Isolierkörper, so dass Temperaturverluste im proximalen Katheterabschnitt 15 beim Durchströmen mit einem Temperierfluid vermieden werden.

Hinsichtlich der Katheterspitze 18 ist vorgesehen, dass diese eine konusförmige Gestalt aufweist. Die konusförmige Gestalt der Katheterspitze 18 kann asymmetrisch sein, so dass eine Durchgangsöffnung 27, die sich durch die Katheterspitze 18 erstreckt, mit dem Durchgangskanal 11 geradlinig fluchtet. Bei dem Ausführungsbeispiel gemäß Fig. 1 weist die Katheterspitze 18 einen Außendurchmesser auf, der höchstens dem Außendurchmesser des distalen Katheterabschnitts 16 entspricht.

Fig. 3 zeigt einen weiteren Querschnitt durch den Katheterschlauch 10 gemäß Fig. 1. Dargestellt ist ein Querschnitt durch den distalen Katheterabschnitt 16 mit dem Wärmetauscherelement 14. Es ist gut erkennbar, dass die Außenwand 20 im proximalen Katheterabschnitt 15 einen größeren Außendurchmesser als die Außenwand 20 im distalen Katheterabschnitt 16 aufweist. Konkret bildet die Außenwand 20 im proximalen Katheterabschnitt 15 eine zylinderförmige proximale Außenumfangsebene 28. Im distalen Katheterabschnitt 16 bildet die Außenwand 20 eine distale Außenumfangsebene 29. Zwischen der proximalen Außenumfangsebene 28 und der distalen Außenumfangsebene 29 ist ein ringförmiger Aufnahmeraum 17 gebildet. Innerhalb des Aufnahmeraums 17, der radial innen durch die distale Außenumfangsebene 29 und radial außen durch die proximale Außenumfangsebene 28 begrenzt ist, ist das komprimierte bzw. gefaltete Wärmetauscherelement 14 angeordnet. Mit anderen Worten kann das Wärmetauscherelement 14 bzw. der Ballon derart komprimierbar bzw. faltbar sein, dass das Wärmetauscherelement 14 vollständig innerhalb des Aufnahmeraums 17 anordenbar ist.

Es ist zweckmäßig, wenn das Wärmetauscherelement 14 im komprimierten bzw. gefalteten Zustand nicht über die proximale Außenumfangsebene 28 radial nach außen vorragt. Vielmehr bildet die proximale Außenumfangsebene 28 der Außenwand 20 im proximalen Katheterabschnitt 15 den größten Querschnittsdurchmesser des gesamten Katheterschlauchs 10. Der komprimierte bzw. gefaltete Zustand wird vorzugweise durch das Aufbringen von äußeren Kräften erreicht bzw. gehalten. Beispielsweise kann eine Schleuse, in der der Katheterschlauch 10 angeordnet ist, eine Begrenzung bilden, die eine Expansion des Wärmetauscherelements 14, insbesondere eines Ballons, verhindert.

Im Hinblick auf das Wärmetauscherelement 14 ist bevorzugt vorgesehen, dass dieses durch einen expandierbaren Ballon gebildet ist. Der Ballon kann einerseits im komprimierten Zustand gefaltet sein und bei Befüllung mit einem Temperierfluid zu seiner Nominalgröße aufgedehnt werden. Ein derartiger Ballon wird im aufgeweiteten bzw. expandierten Zustand hergestellt und durch Zusammenfalten komprimiert. Der Ballon weist daher ein "nicht-compliant"-Verhalten auf. Dabei erhöht sich der Querschnittsdurchmesser des Ballons bei einem relativen Innendruck von 2 bar um höchstens 10%, insbesondere höchstens 5%, insbesondere höchstens 2,5%, insbesondere höchstens 1%, gegenüber dem voll expandierten Zustand des Ballons (relativer Innendruck ca. 0,1 bar bis 0,2 bar). Alternativ kann ein so genannter Compliant-Ballon eingesetzt werden, der ein elastisches Material umfasst. Derartige Ballone werden im komprimierten Zustand hergestellt. Der elastische Ballon wird durch einen Fluiddruck radial aufgedehnt und schrumpft bei Nachlassen des Fluiddrucks auf die ursprüngliche Nominalgröße zusammen.

Generell ist das Wärmetauscherelement 14 bzw. der Ballon mit den Temperierkanälen 12, 13 fluidverbunden. Dazu weist die Außenwand 20 vorzugsweise zwei Fluidöffnungen 33, 34 auf, wobei jede der beiden Fluidöffnungen 33, 34 jeweils einem Temperierkanal 12, 13 zugeordnet ist. Beide Fluidöffnungen 33, 34 münden in den Ballon bzw. das Wärmetauscherelement 14. Insgesamt ergibt sich auf diese Weise ein Temperierkreislauf. Dabei kann ein erster Temperierkanal 12 eine Fluidzufuhrleitung und ein zweiter Temperierkanal 13 eine Fluidrückführleitung bilden. Temperierfluid, vorzugsweise ein Kühlmedium, kann über den ersten Temperierkanal 12 zum Wärmetauscherelement 14 bzw. Ballon strömen. Das Temperierfluid durchströmt den Ballon, vorzugsweise von distal nach proximal und gelangt über eine proximale Fluidöffnung 34 in der Außenwand 20 in den zweiten Temperierkanal 13. Über den zweiten Temperierkanal 13 bzw. die Fluidrückführleitung wird das Temperierfluid aus dem Katheterschlauch 10 herausgeleitet.

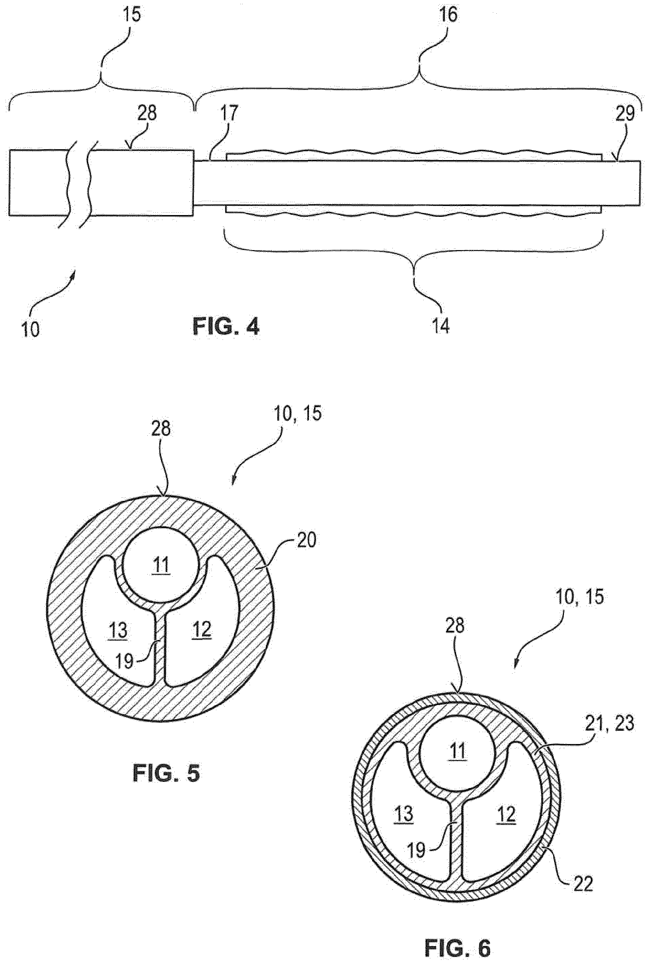

In Fig. 4 ist das Prinzip der Erfindung nochmals schematisch dargestellt. Der vereinfacht dargestellte Katheterschlauch 10 weist im Allgemeinen einen proximalen Katheterabschnitt 15 und einen distalen Katheterabschnitt 16 auf. Im distalen Katheterabschnitt 16 ist ein gefalteter Ballon als Wärmetauscherelement 14 angeordnet. Der Ballon bzw. das Wärmetauscherelement 14 wird vom distalen Katheterabschnitt 16 des Katheterschlauchs 10 getragen. Konkret ist das Wärmetauscherelement 14 im gefalteten Zustand derart angeordnet, dass es innerhalb eines Aufnahmeraums 17 angeordnet ist, der in radialer Richtung einerseits durch die proximale Außenumfangsebene 28 und andererseits durch die distale Außenumfangsebene 29 begrenzt ist.

Der Katheterschlauch 10 kann grundsätzlich einteilig bzw. einstückig hergestellt sein. Dies kann einerseits durch ein entsprechendes Extrusionsverfahren erfolgen. Andererseits kann der Katheterschlauch 10 mit einer einheitlichen, relativ größeren Wandstärke der Außenwand 20 hergestellt werden. In einem Nachbearbeitungsschritt kann dann durch ein mechanisches oder lasergestütztes Abtragverfahren im distalen Kathteterabschnitt 16 die Wandstärke vom Außenumfang ausgehend reduziert werden, so dass der distale Katheterabschnitt 16 schlussendlich kleinere Außendimensionen aufweist als der proximale Katheterabschnitt 15.

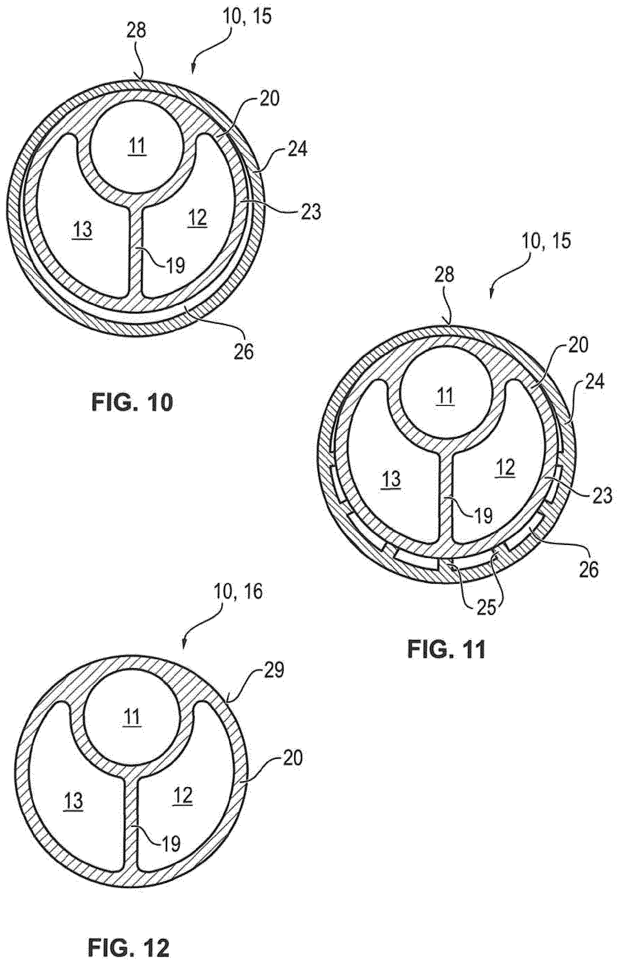

Fig. 5 zeigt am Beispiel eines Katheterschlauchs 10, der einteilig ausgebildet ist, einen Querschnitt durch den proximalen Katheterabschnitt 15, wobei erkennbar ist, dass der proximale Katheterabschnitt 15 eine relativ große Wandstärke aufweist. Fig. 12 zeigt demgegenüber einen Querschnitt durch denselben Katheterschlauch 10 im distalen Katheterabschnitt 16. Es ist gut erkennbar, dass die Außenwand 20 des Katheterschlauchs 10 im distalen Katheterabschnitt 16 eine kleinere Wandstärke aufweist als im proximalen Katheterabschnitt 15. Die Dimensionen der Kanäle 11, 12, 13 sind vorzugsweise identisch. Mit anderen Worten weist der Katheterschlauch 10 insgesamt einen konstanten Innendurchmesser auf. Lediglich der Außendurchmesser des Katheterschlauchs 11 variiert, wobei damit eine Variation der Wandstärke der Außenwand 20 einhergeht.

Durch die verringerte Wandstärke der Außenwand 20 im distalen Katheterabschnitt 16 ist der distale Katheterabschnitt 16 biegeflexibler als der proximale Katheterabschnitt 15. Dies ermöglicht die Zuführung des Katheterschlauchs 10 in kleine und eng gewundene Blutgefäße. Eine verbesserte Zuführbarkeit in kleine und eng gewundene Blutgefäße, insbesondere eine gute Biegeflexibilität des Katheterschlauchs 10, ergibt sich auch bei der bevorzugten Ausgestaltung, bei der der distale Katheterabschnitt 16 eine Skalierung des proximalen Katheterabschnitts 15 ist, d.h. wenn im distalen Katheterabschnitt 16 auch die Querschnittsflächen der Temperierkanäle 12, 13 kleiner als im proximalen Katheterabschnitt 15 sind.

Die erhöhte Wandstärke der Außenwand 20 im proximalen Katheterabschnitt 15 versteift den proximalen Katheterabschnitt 15, so dass sich der Katheterschlauch 11 insgesamt gut längsaxial schieben lässt. Eine am proximalen Axialende aufgebrachte Vorschubkraft wird so gut an die Katheterspitze 18 übertragen. Gleichzeitig bewirkt die erhöhte Wandstärke der Außenwand 20 im proximalen Katheterabschnitt 15 eine verbesserte Isolationswirkung, so dass Temperaturverluste beim Durchströmen der Temperierkanäle 12, 13 im proximalen Katheterabschnitt 15 vermieden werden. Dies erhöht den Wirkungsgrad der lokalen Temperierung bzw. Kühlung am Wärmetauscherelement 14. Bei der Variante des Katheters, bei der der Katheterschlauch 10 einstückig, beispielsweise über einen Extrusionsprozess, hergestellt ist, werden vorzugsweise Materialien wie Polyurethan, Pebax, Nylon, thermoplastische Kunsstoffe, insbesondere thermoplastische Elastomere, eingesetzt. PTFE oder andere Fluorpolymere sind insofern vorteilhaft, da auf diese Weise die Reibung gegenüber einer Schleuse und/oder der Gefäßwand reduziert wird. Allerdings erschweren diese Materialien die Beklebung mit dem Wärmetauscherelement 14 bzw. Ballon. Insofern sind Nylon oder PET als Material für den Ballon zweckmäßig.

Anstelle einer einteiligen Ausbildung des Katheterschlauchs 10 kann dieser auch mehrteilig ausgebildet sein. Insbesondere kann der Katheterschlauch 10 einen Innenschlauch 23 umfassen, der sich vollständig durch den proximalen Katheterabschnitt 15 und den distalen Katheterabschnitt 16 erstreckt. Der Innenschlauch 23 kann einen konstanten Querschnitt aufweisen, insbesondere in Form des Querschnitts gemäß Fig. 12. Um den Außendurchmesser im proximalen Katheterabschnitt 15 zu erhöhen und somit eine Durchmesserdifferenz zwischen dem proximalen Katheterabschnitt 15 und dem distalen Katheterabschnitt 16 bereitzustellen, kann der Innenschlauch 23 im proximalen Katheterabschnitt 15 mit einer zusätzlichen Schicht versehen sein. Konkret kann vorgesehen sein, dass der Katheterschlauch 10 bzw. der Außenwand 20 im proximalen Katheterabschnitt 15 mehrschichtig aufgebaut ist, wobei wenigstens eine innere Schicht 21 und eine äußere Schicht 23 vorgesehen sind.

Die innere Schicht 21 wird vorzugsweise durch den Innenschlauch 23 gebildet. Die äußere Schicht 22 kann durch einen Schrumpfschlauch, eine vollflächige Beschichtung, insbesondere ein Coating, oder durch einen weiteren Schlauch gebildet sein, der mit dem Innenschlauch 23 beispielsweise durch Laminieren verbunden ist. Jedenfalls besteht zwischen der inneren Schicht 21 und der äußeren Schicht 22 ein vollflächiger, vorzugsweise stoffschlüssiger, Kontakt. Die äußere Schicht 22 und die innere Schicht 21 können aus verschiedenen Materialien gebildet sein.

Die innere Schicht 21 und die äußere Schicht 22 können durch ein Koextrusionsverfahren gleichzeitig hergestellt werden. Alternativ kann zunächst der Innenschlauch 23 bzw. die innere Schicht 21 hergestellt werden und anschließend die äußere Schicht 22 auf der inneren Schicht 21 im proximalen Katheterabschnitt 15 angebracht werden. Die äußere Schicht 22 kann Füllstoffe, Verstärkungsstoffe oder andere Additive aufweisen, um der äußeren Schicht 22 andere Eigenschaften als der inneren Schicht 21 zu verleihen. Insbesondere kann die äußere Schicht 22 eine Verstärkungsschicht bilden, um die längsaxiale Stabilität der Außenwand 20 im proximalen Katheterabschnitt 15 zu erhöhen. Beispielsweise kann die äußere Schicht 22 nanoverstärkte Verbundwerkstoffe aufweisen, die beispielsweise Nylon, thermoplastische Elastomere oder thermoplastische Polyurethane umfassen. Ferner kann durch Verwendung eines Materials für die äußere Schicht 22, das härter als das Material der inneren Schicht 21 ist, eine Verstärkung des proximalen Katheterabschnitts 15 erreicht werden. Die äußere Schicht 22 kann auch ein Drahtgeflecht (Braiding) oder einen spiralförmig gewundenen Draht (Coiling) als Verstärkungselement umfassen.

Für eine verbesserte thermische Isolierung ist es bevorzugt, wenn die äußere Schicht 22 ein Material mit geringer Wärmeleitfähigkeit aufweist. Insbesondere kann vorgesehen sein, dass die äußere Schicht eine Wärmeleitfähigkeit aufweist, die höchstens 0,2 Wm⁻¹K⁻¹, insbesondere höchstens 0,15 Wm⁻¹K⁻¹, insbesondere höchstens 0,1 Wm⁻¹K⁻¹, insbesondere höchstens 0,08 Wm⁻¹K⁻¹, beträgt.

Generell kann die äußere Schicht 22 als zusätzlicher Schlauch, insbesondere als Außenschlauch 24 ausgebildet sein. Der Außenschlauch 24 kann einen Innendurchmesser aufweisen, der größer als ein Außendurchmesser des Innenschlauchs 23 ist. Vorzugsweise wird der Außenschlauch 24 im Bereich des proximalen Katheterabschnitts 15 über den Innenschlauch 23 geschoben und zumindest an einem distalen Axialende mit dem Innenschlauch 23 verbunden. Beispielweise kann das distale Axialende des Außenschlauchs 24 geschrumpft oder gecrimpt werden, um mit dem Innenschlauch 23 eine fluiddichte Verbindung zu schaffen. Der Außenschlauch 24 kann mit dem Innenschlauch 23 auch durch Kleben oder Schweißen verbunden sein.

Das gecrimpte Axialende kann einen Abstandshalter 25 bilden, der einen Spalt bzw. Isolierraum 26 zwischen dem Außenschlauch 24 und dem Innenschlauch 23 offenhält. Andere Arten von Abstandshaltern 25 sind möglich. Insbesondere können entlang des Außenschlauchs 24 mehrere Abstandshalter 25 angeordnet sein, die einstückig mit dem Außenschlauch 24 oder dem Innenschlauch 23 verbunden oder separat, beispielsweise als Ring, zwischen dem Außenschlauch 24 und dem Innenschlauch 23 angeordnet sind. Unabhängig von der Art und Gestaltung der Abstandshalter 25 ist vorgesehen, dass der Isolierraum 26 eine radiale Höhe, also der Außenschlauch 24 und der Innenschlauch 23 einen Abstand zueinander aufweisen, der mindestens 50µm, insbesondere mindestens 100µm, insbesondere mindestens 150µm, beträgt.

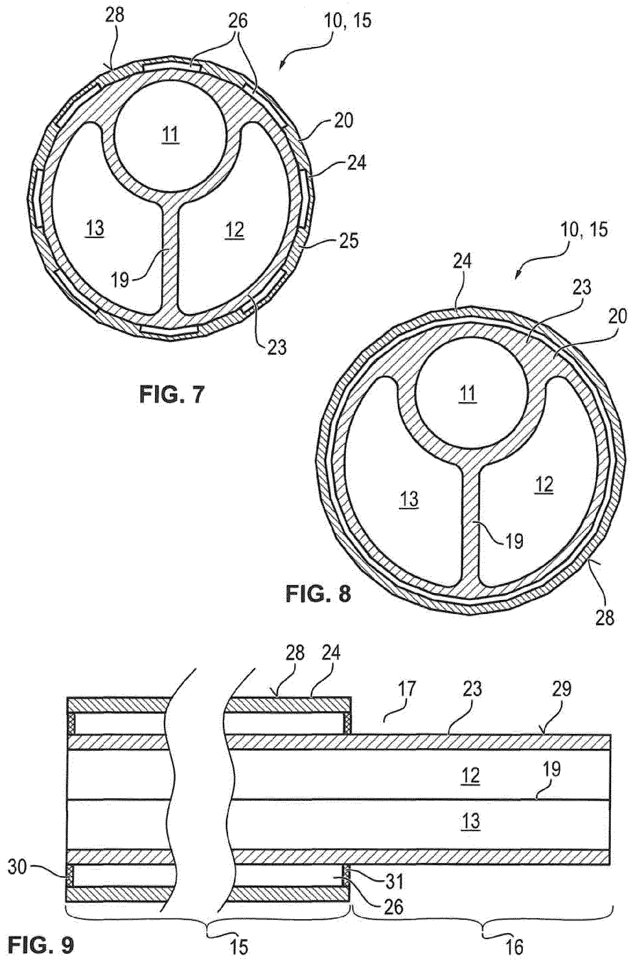

Fig. 7 zeigt ein Ausführungsbeispiel, bei dem der Katheterschlauch 10 im proximalen Katheterabschnitt 15 radial nach innen vorstehende Abstandshalter 25 aufweist, so dass zwischen dem Außenschlauch 24 und dem Innenschlauch 23 ein Isolierraum 26 ausgebildet ist. Vorzugsweise erstrecken sich über den Umfang des Innenschlauchs 23 mehrere Abstandshalter 25. Die Abstandshalter 25 können einerseits als radial nach innen vorspringende Elemente des Außenschlauchs 24 und andererseits als nach außen radial vorstehende Elemente des Innenschlauchs 23 ausgebildet sein. Der Isolierraum 26 bildet vorzugsweise einen Luftraum, der zu einer verbesserten Isolierung der Außenwand 20 im proximalen Katheterabschnitt 15 beiträgt. Generell können zumindest im Bereich der Temperierkanäle 12, 13 Abstandshalter 25 zwischen dem Innenschlauch 23 und dem Außenschlauch 24 angeordnet sein. Bei dem Ausführungsbeispiel gemäß Fig. 7 sind über den gesamten Umfang des Innenschlauchs 23 Abstandshalter 25 angeordnet, so dass der Außenschlauch 24 und der Innenschlauch 23 koaxial zueinander beabstandet angeordnet sind, um den Isolierraum 26 zu bilden.

Der Isolierraum 26 kann auch ohne den Einsatz von Abstandshaltern 25 gebildet sein. Ein derartiges Ausführungsbeispiel zeigt Fig. 8. Konkret ist in Fig. 8 ein Querschnitt durch einen proximalen Katheterabschnitt 15 eines Katheterschlauchs 10 gezeigt, wobei die Außenwand 20 des Katheterschlauchs 10 im proximalen Katheterabschnitt 15 durch einen Innenschlauch 23 und einen Außenschlauch 24 gebildet ist. Der Außenschlauch 24 und der Innenschlauch 23 sind koaxial zueinander angeordnet und voneinander radial beabstandet. Auf diese Weise ist zwischen dem Außenschlauch 24 und dem Innenschlauch 23 ein ringförmiger Isolierraum 26 gebildet.

In Fig. 9 ist ein Längsschnitt durch den Katheterschlauch 10 gemäß Fig. 8 gezeigt. Der Katheterschlauch 10 umfasst einen proximalen Katheterabschnitt 15 und einen distalen Katheterabschnitt 16. Der Innenschlauch 23 erstreckt sich über die gesamte Länge des Katheterschlauchs 10, insbesondere durch den proximalen Katheterabschnitt 15 und den distalen Katheterabschnitt 16. Der Außenschlauch 24 erstreckt sich ausschließlich entlang des proximalen Katheterabschnitts 15. Der Außenschlauch 24 bildet eine proximale Außenumfangsebene 28, die radial außen einen im distalen Katheterabschnitt 16 angeordneten Aufnahmeraum 17 begrenzt. Der Aufnahmeraum 17 ist radial innen durch eine distale Außenumfangsebene 29 begrenzt, die durch den Innenschlauch 23 definiert wird. Der Außenschlauch 24 ist koaxial zum Innenschlauch 23 angeordnet, wobei zwischen dem Außenschlauch 24 und dem Innenschlauch 23 ein Abstand besteht. Der Abstand zwischen dem Außenschlauch 24 und dem Innenschlauch 23 wird durch einen Isolierraum 26 gefüllt. Um den Isolierraum 26 abzuschließen, ist am proximalen Ende des Katheterschlauchs 10 ein proximaler Abschlussflansch 30 vorgesehen. Am distalen Ende des proximalen Katheterabschnitts 15 ist ein distaler Abschlussflansch 31 vorgesehen. Die Abschlussflansche 30, 31 sind im Wesentlichen ringförmig ausgebildet und schließen den Isolierraum 26 fluiddicht in distaler und proximaler Richtung ab.

Dazu verbinden die Abschlussflansche 30, 31 den Innenschlauch 23 mit dem Außenschlauch 24.

In Fig. 10 ist ein weiteres Ausführungsbeispiel des erfindungsgemäßen medizinischen Katheters gezeigt. Dargestellt ist insbesondere ein Querschnitt durch den Katheterschlauch 10 im proximalen Katheterabschnitt 15. Der Katheterschlauch 10 weist im proximalen Katheterabschnitt 15 einen Innenschlauch 23 und einen Außenschlauch 24 auf, wobei der Außenschlauch 24 exzentrisch zum Innenschlauch 23 angeordnet ist. Insbesondere liegt der Außenschlauch 24 im Bereich des Durchgangskanals 11 bzw. des Instrumentenlumens am Innenschlauch 23 an. Vorzugsweise ist der Außenschlauch 24 an dieser Stelle mit dem Innenschlauch 23 fest verbunden, beispielsweise verschweißt oder verklebt. Im Bereich der Temperierkanäle 12, 13 weist der Außenschlauch 24 hingegen einen Abstand zum Innenschlauch 23 auf. Auf diese Weise ist zumindest im Bereich der Temperierkanäle 12, 13 zwischen dem Außenschlauch 24 und dem Innenschlauch 23 ein Isolierraum 26 gebildet.

Um zu gewährleisten, dass zwischen dem Außenschlauch 24 und dem Innenschlauch 23 ein Abstand, insbesondere ein Isolierraum 26, besteht, können zumindest im Bereich der Temperierkanäle 12, 13 zwischen dem Außenschlauch 24 und dem Innenschlauch 23 Abstandshalter 25 angeordnet sein. Ein derartiges Ausführungsbeispiel, bei dem der Außenschlauch 24 exzentrisch zum Innenschlauch 23 angeordnet und im Bereich der Temperierkanäle 12, 13 durch Abstandshalter 25 von dem Innenschlauch 23 auf Abstand gehalten wird, ist in Fig. 11 gezeigt. Analog zu dem Ausführungsbeispiel gemäß Fig. 7 können die Abstandshalter 25 sowohl einstückig mit dem Außenschlauch 24, als auch einstückig mit dem Innenschlauch 23 verbunden bzw. geformt sein. Jedenfalls besteht zwischen dem Innenschlauch 23 und dem Außenschlauch 24 im Bereich der Temperierkanäle 12, 13 ein Isolierraum 26. Dabei ist es nicht erforderlich, mehrere Abstandshalter 25 vorzusehen. Vielmehr kann ein einzelner Abstandhalter 25 ausreichen, um einen beständigen Abstand zwischen dem Außenschlauch 24 und dem Innenschlauch 23 bereitzustellen. Vorzugsweise ist der einzige Abstandshalter 25 (radial) gegenüber einer Verbindungsstelle zwischen dem Außenschlauch 24 und dem Innenschlauch 23 angeordnet.

Fig. 12 zeigt einen Querschnitt durch den Katheterschlauch 10 im distalen Katheterabschnitt 16. Der Katheterschlauch 10 weist im distalen Katheterabschnitt 16 ebenfalls einen Durchlasskanal 11 und zwei Temperierkanäle 12, 13 auf, die durch eine Innenwand 19 voneinander getrennt sind. Der Katheterschlauch 10 umfasst ferner eine Außenwand 20, die eine relativ kleine Wandstärke aufweist. Insbesondere ist die Außenwand 20 im distalen Katheterabschnitt 16 einschichtig ausgebildet. Die Außenwand 20 bildet eine distale Außenumfangsebene 29 des Katheterschlauchs 10.

Für alle Ausführungsbeispiele gemäß den Fig. 5 bis 11, die jeweils den proximalen Katheterabschnitt 15 zeigen, gilt, dass der entsprechende bzw. zugehörige distale Katheterabschnitt 16 eine Geometrie gemäß Fig. 12 aufweisen kann. Mit anderen Worten zeigt Fig. 12 einen distalen Katheterabschnitt 16 des Katheterschlauchs 10 gemäß einem der Fig. 5 bis 11. Dabei kann der distale Katheterabschnitt 16 gemäß Fig. 12 einstückig mit dem proximalen Katheterabschnitt 15 gemäß Fig. 5 ausgebildet sein. Alternativ kann eines der Ausführungsbeispiele gemäß Fig. 6 bis 11 mit einem Innenschlauch 23 ausgebildet sein, der eine Querschnittsgeometrie gemäß Fig. 12 aufweist und sich über den proximalen Katheterabschnitt 15 hinaus erstreckt, um den distalen Katheterabschnitt 16 zu bilden.

Im Allgemeinen kann vorgesehen sein, dass bei den Ausführungsbeispielen gemäß Fig. 6 bis 11 die äußere Schicht 22 bzw. der Außenschlauch 24 derart gestaltet sein können, dass die Wärmeleitfähigkeit reduziert ist. Beispielsweise kann die äußere Schicht 22 bzw. der Außenschlauch 24 eine Porosität aufweisen, insbesondere eine schaumartige Struktur besitzen. Dies kann beispielsweise durch Einbringen von Mikroblasen während des Extrusionsprozesses erreicht werden. Alternativ können in das Material des Außenschlauchs 24 bzw. der äußeren Schicht 22 hohle Mikrokugeln eingebracht werden. Insgesamt ist es bevorzugt, wenn der Außenschlauch 24 bzw. die äußere Schicht 22 Hohlräume aufweist, in der Luft aufgenommen werden kann. Damit wird die Isolationswirkung der äußeren Schicht 22 bzw. des Außenschlauchs 24 verbessert. Alternativ können Elastomere, isolierende Schaumschichten aus vernetzbaren Copolymeren zur Isolierung verwendet werden. Vorzugsweise werden derartige Materialien in die äußere Schicht 22 bzw. den Außenschlauch 24 integriert. Die elastomeren, isolierenden Schaumschichten können beispielsweise Polyethylen- und Polypropylen-Einheiten oder Polyethylen-Propylen-Dien beinhalten.

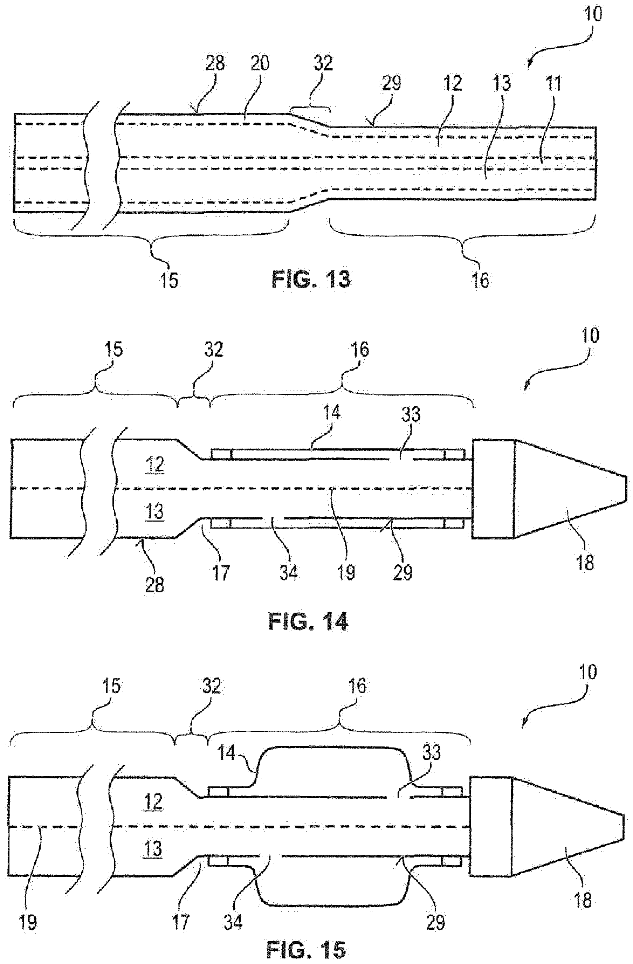

In Fig. 13 ist ein alternatives Ausführungsbeispiel des erfindungsgemäßen Katheters gezeigt. Im Unterschied zu den vorhergehenden Ausführungsbeispielen wird bei dem Ausführungsbeispiel gemäß Fig. 13 nicht die Wandstärke entlang des Katheterschlauchs 10 variiert, sondern vielmehr der Innenquerschnittsdurchmesser des Katheterschlauchs 10 im distalen Katheterabschnitt 16 gegenüber dem proximalen Katheterabschnitt 15 reduziert. Konkret können Temperierkanäle 12, 13 im distalen Katheterabschnitt 16 eine kleinere Querschnittsfläche als im proximalen Katheterabschnitt 15 aufweisen. Die Wandstärke der Außenwand 20 kann hingegen entlang des gesamten Katheterschlauchs 10 konstant sein. Ebenfalls ist vorzugsweise die Querschnittsfläche des Durchgangskanals 11 entlang des gesamten Katheterschlauchs 10 konstant. Es ist allerdings auch möglich, dass sowohl die Wandstärke, als auch die Querschnittsflächen der einzelnen Kanäle 11, 12, 13 entlang des Katheterschlauchs 10 variieren. Die Temperierkanäle 12, 13 können im proximalen Katheterabschnitt 15 eine größere Querschnittsfläche als im distalen Katheterabschnitt 16 aufweisen. So kann die Menge an Temperierfluid, die durch den Temperierkreislauf geführt werden kann, erhöht werden. Dies optimiert die Wärmetauscherfunktion des gesamten Katheters. Um die Zuführbarkeit des Katheters bzw. Katheterschlauchs 10 zu verbessern, ist vorgesehen, dass zwischen dem proximalen Katheterabschnitt 15 und dem distalen Katheterabschnitt 16 ein konusförmiger Übergangsabschnitt 32 ausgebildet ist. Die Länge des konusförmigen Übergangsabschnitts 32 beträgt vorzugsweise höchstens 120 mm, insbesondere höchstens 100 mm, insbesondere höchstens 80 mm, insbesondere höchstens 60 mm, insbesondere höchstens 40 mm, insbesondere höchstens 30 mm, insbesondere höchstens 20 mm. Im Minimum beträgt die Länge des konusförmigen Übergangsabschnitts 32 vorzugsweise 10 mm.

In den Fig. 14 und 15 ist ein weiteres alternatives Ausführungsbeispiel für einen erfindungsgemäßen Katheter gezeigt. Der Katheterschlauch gemäß Fig. 14 und 15 weist einen proximalen Katheterabschnitt 15 und einen distalen Katheterabschnitt 16 auf. Der proximale Katheterabschnitt 15 weist einen größeren Außendurchmesser als der distale Katheterabschnitt 16 auf. Im distalen Katheterabschnitt 16 trägt der Katheterschlauch 10 ein Wärmetauscherelement 14. Im vorliegenden Fall ist das Wärmetauscherelement 14 als Compliant-Ballon ausgebildet. Mit anderen Worten ist der Ballon elastisch, sodass er sich unter Fluiddruck radial aufdehnt.

Im Längsschnitt durch den Katheterschlauch 10 ist gemäß Fig. 14 und 15 der Temperierkreislauf erkennbar. Der Temperierkreislauf umfasst den ersten Temperierkanal 12, eine distale Fluidöffnung 32 in der Außenwand 20, das Wärmetauscherelement 14 bzw. den Compliant-Ballon, eine proximale Fluidöffnung 34 in der Außenwand 20 und den zweiten Temperierkanal 13.

Der Ballon bzw. das Wärmetauscherelement 14 ist fluiddicht mit der Außenwand 20 verbunden, so dass Temperierfluid den Ballon von distal nach proximal, also von der distalen Fluidöffnung 33 zur proximalen Fluidöffnung 34, durchströmen kann. Am distalen Axialende des Katheterschlauchs 10 ist eine Katheterspitze 18 montiert, die die Temperierkanäle 12, 13 fluiddicht verschließt. Die Katheterspitze 18 weist eine Durchgangsöffnung 27 auf, die mit dem Durchgangskanal 11 verbunden ist, so dass ein Instrument, beispielsweise ein Führungsdraht, durch den Katheterschlauch 10 an den Behandlungsort geführt werden kann.

Der Compliant-Ballon bzw. das Wärmetauscherelement bildet im Ruhezustand einen zylinderförmigen Schlauch, der, wie in Fig. 14 dargestellt ist, innerhalb des Aufnahmeraums 17 zwischen der proximalen Außenumfangsebene 28 und der distalen Außenumfangsebene 29 angeordnet ist. Der Aufnahmeraum 17 ist bei dem Ausführungsbeispiel gemäß Fig. 14 und 15 in längsaxialer Richtung einerseits durch den Übergangsabschnitt 32 und andererseits durch die Katheterspitze 18 begrenzt. Die Katheterspitze 18 weist vorzugsweise einen Außendurchmesser auf, der dem Außendurchmesser des proximalen Katheterabschnitts 15 entspricht. Auf diese Weise kommt der Ballon bzw. das Wärmetauscherelement 14 beim Zuführen des Katheterschlauchs 10 durch eine Schleuse oder durch ein Blutgefäß weder mit einer Schleusenwand noch mit einer Gefäßwand in Kontakt. So entsteht zwischen einer Schleusenwand oder einer Gefäßwand und dem Wärmetauscherelement 14 keine Reibung, was die Zufuhr des Katheterschlauchs 10 insgesamt erleichtert. Konkret kann auf eine reibungshemmende bzw. reibungsreduzierende Beschichtung des Ballons bzw. des Wärmetauscherelements 14 verzichtet werden, die anderenfalls die Wärmeübertragung über das Wärmetauscherelement 14 beeinträchtigen kann. Das Wärmetauscherelement 14 weist daher ein verbessertes Wärmeübertragungsverhalten auf.

Fig. 15 zeigt das Ausführungsbeispiel gemäß Fig. 14, wobei der Ballon bzw. das Wärmetauscherelement 14 den expandierten Zustand eingenommen hat. Der expandierte Zustand wird durch den Fluiddruck erreicht, wenn Temperierfluid, insbesondere ein Kühlmedium, durch die Temperierkanäle 12, 13 und das Wärmetauscherelement 14 strömt. Der Fluiddruck bewirkt ein Aufdehnen des Compliant-Ballons, so dass die Wärmeübertragungsfläche vergrößert wird. Dies erhöht weiterhin die Wirksamkeit des erfindungsgemäßen Katheters als Wärmetauscher bzw. für die hypothermische Behandlung.

Der erfindungsgemäße Katheter kann insbesondere zur lokalen Blutkühlung für zerebrale Blutgefäße eingesetzt werden. Eine derartige therapeutische Hypothermie kann insbesondere zur Behandlung eines Schlaganfalls eingesetzt werden. Vorzugsweise wird der Katheter mit einem Rekanalisationselement, beispielsweise einem Thrombenfänger, kombiniert. Das Rekanalisationselement kann während der hypothermischen Behandlung durch den Durchgangskanal 11 an einen Thrombus geführt werden, den Thrombus greifen und entfernen.

Im Sinne einer verbesserten Biegeflexibilität bei gleichzeitig hoher Wärmeaustauschwirkung und guter Zuführbarkeit des Katheters schlägt die Erfindung allgemein vor, den Querschnittsdurchmesser des Katheterschlauchs 10 im distalen Katheterabschnitt 16 kleiner als im proximalen Katheterabschnitt 15 zu gestalten. Dadurch wird der proximale Katheterabschnitt 15 verstärkt, was zu einer verbesserten Schiebbarkeit beim Zuführen des Katheters führt. Eine erhöhte Wandstärke im proximalen Katheterabschnitt 15 verbessert außerdem die Isolationswirkung der Außenwand 20. Eine geringere Wandstärke im distalen Katheterabschnitt 16 führt zu einer höheren Flexibilität. Generell kann der Katheterschlauch 10 in weitere Unterbereiche unterteilt werden, wobei Kathetergeometrie, Wandstärke, Materialeigenschaften und/oder die Porosität der äußeren Schicht 22 bzw. des Außenschlauchs 24 variieren können. Hinsichtlich der Dimensionen des Katheters gilt im Allgemeinen:
Bei einem Außendurchmesser des proximalen Katheterabschnitts 15 von mehr als 2 mm beträgt die Länge des Katheterschlauchs 10 vorzugsweise wenigstens 80 cm, insbesondere wenigstens 90 cm, insbesondere wenigstens 100 cm, insbesondere wenigstens110 cm, insbesondere wenigstens 120 cm. Die Länge des Katheterschlauchs 10 ist vorzugsweise höchstens 140 cm bei einem Außendurchmesser des proximalen Katheterabschnitts 15 von mehr als 2 mm.

Bei einem Außendurchmesser des proximalen Katheterabschnitts 16 von weniger als 2 mm beträgt die Länge des Katheterschlauchs 10 vorzugsweise wenigstens 80 cm, insbesondere wenigstens 90 cm, insbesondere wenigstens 100 cm, insbesondere wenigstens 110 cm, insbesondere wenigstens 120 cm, insbesondere wenigstens 130 cm, insbesondere wenigstens 140 cm. Die Länge des Katheterschlauchs 10 bei einem Außendurchmesser des proximalen Katheterabschnitts 15 von weniger als 2 mm beträgt vorzugsweise maximal 160 cm.

Die Wandstärke des Außenschlauchs 24 beträgt vorzugsweise zwischen 100µm und 300µm, vorzugsweise zwischen 150µm und 250µm. Falls die Außenwand 20 im proximalen Katheterabschnitt 15 mit einer äußeren Schicht 22 versehen ist, weist diese vorzugsweise eine Schichtdicke zwischen 50µm und 150µm, insbesondere zwischen 70µm und 130µm, insbesondere zwischen 90µm und 100µm, auf.

Insgesamt gilt für bevorzugte Ausführungsbeispiele der Erfindung, insbesondere bei einem einstückig ausgebildeten Katheterschlauch, dass die Differenz des Außendurchmessers zwischen den proximalen Kathterabschnitt und dem distalen Katheterabschnitt zwischen 100 µm und 1000 µm, insbesondere zwischen 200 und 800 µm, insbesondere zwischen 300 µm und 700 µm, insbesondere zwischen 400 und 600 µm, vorzugsweise insbesondere 500 µm, betragen kann. Dabei wird der jeweilige dreilumige Teilbereich (mit drei Kanälen) betrachtet (nicht die Spitze, falls diese aus einem einzigen Kanal besteht).

Für eine gute Schiebbarkeit des Katheterschlauchs 10 ist der flexible distale Katheterabschnitt 16 vorzugweise längenbegrenzt. Insbesondere kann der distale Katheterabschnitt 16 eine Länge aufweisen, die höchstens 120 mm, insbesondere höchstens 100 mm, insbesondere höchstens 80 mm, insbesondere höchstens 60 mm, beträgt. Vorzugsweise ist der distale Katheterabschnitt 16 jedoch länger als 20 mm.

Ein Ausführungsbeispiel der Erfindung weist folgende technische Spezifikationen auf:
- Der Katheterschlauch 10 eignet sich zur Zuführung durch eine Schleuse der Größe 8 French (Innendurchmesser der Schleuse ca. 2,8 mm);
- Der Außendurchmesser des proximalen Katheterabschnitts 15 beträgt zwischen 2,3 mm und 2,7 mm, bevorzugt zwischen 2,5 mm und 2,7 mm, insbesondere konkret 2,6 mm;
- Der Außendurchmesser des distalen Katheterabschnitts 16 beträgt zwischen (ohne Wärmetauscherelement/Ballon) 1,9 mm und 2,3 mm, bevorzugt zwischen 2,0 mm und 2,2 mm, insbesondere konkret 2,1 mm;
- Die Gesamtlänge des Katheterschlauchs 10 beträgt zwischen 1,0 m und 1,2 m, bevorzugt 1,1 m;
- Die Länge des distalen Katheterabschnitts 16 beträgt zwischen 40 mm und 120 mm, insbesondere zwischen 60 mm und 100 mm, vorzugsweise zwischen 70 mm und 90 mm, insbesondere konkret 80 mm.

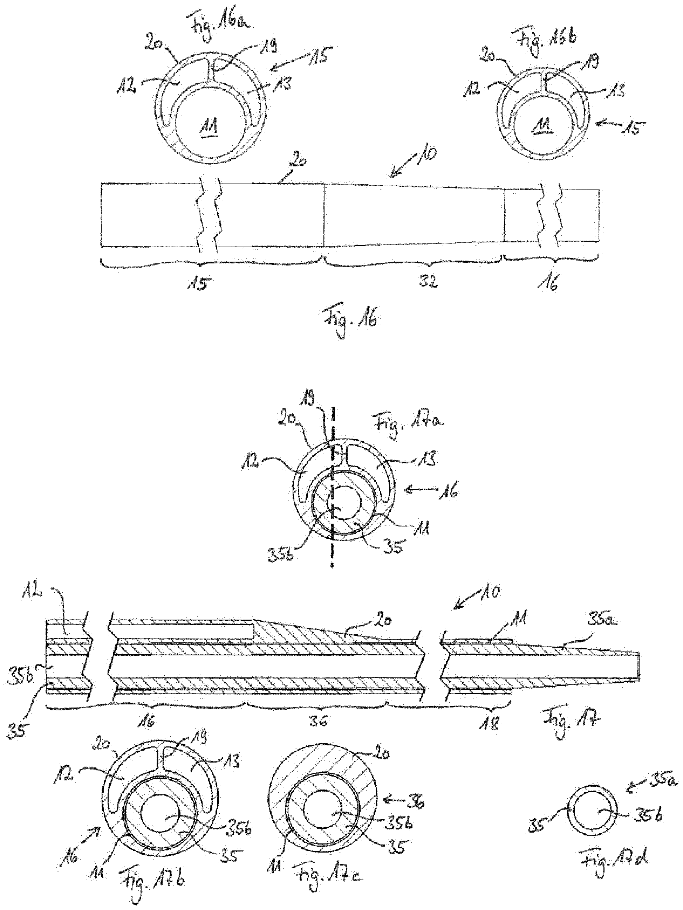

Fig. 16 zeigt einen Längs- und zwei Querschnitte eines erfindungsgemäßen Katheters nach einem bevorzugten Ausführungsbeispiel. Der Katheterschlauch 10 umfasst einen proximalen Katheterabschnitt 15 und einen distalen Katheterabschnitt 16, wobei der distale Katheterabschnitt 16 einen kleineren Außendurchmesser als der proximale Katheterabschnitt 15 aufweist. Das Ausführungsbeispiel gemäß Fig. 16 entspricht im Wesentlichen dem Ausführungsbeispiel gemäß Fig. 13. Allerdings ist bei dem Katheterschlauch 10 gemäß Fig. 16 der distale Katheterabschnitt 16 eine Skalierung des proximalen Katheterabschnitts 15. Mit anderen Worten sind die Querschnitte der beiden Katheterabschnitte 15, 16 zueinander ähnlich im mathematischen bzw. geometrischen Sinn. Insbesondere das Verhältnis zwischen den Querschnittsflächen der Temperierkanäle 12, 13 und der Innenwand 19 ist im proximalen Katheterabschnitt 15 und im distalen Katheterabschnitt 16 identisch. Dies ist in den Fig. 16a und 16b gut erkennbar.

In Fig. 17 ist ein Längsschnitt durch einen distalen Katheterabschnitt 16 eines Katheterschlauch 10 gezeigt. Der Längsschnitt verläuft nicht durch die Längsachse des Katheterschlauchs 10, sondern ist seitlich versetzt (Fig. 17a). Gut erkennbar ist der Durchgangskanal 11. Durch diesen erstreckt sich bei dem dargestellten Ausführungsbeispiel ein Dilatator 35. Der Durchgangskanal 11 weist einen konstanten, gleichbleibenden Querschnittsdurchmesser auf. Der Außendurchmesser der Katheterschlauchs 10 ist hingegen unterschiedlich. Insbesondere weist der Katheterschlauch 10 eine Katheterspitze 18 auf, die sich unmittelbar an den distalen Katheterabschnitt 16 anschließt. Im distalen Katheterabschnitt 16 sind die hier aus Gründen der Übersichtlichkeit nicht dargestellten Wärmetauscherelemente 14 angeordnet. Der distale Katheterabschnitt 16 definiert sich also als Abschnitt nahe der Katheterspitze 18, der den Ballon oder die Ballone trägt.

In dem Katheterschlauch 10 verlaufen neben dem exzentrisch bzw. seitlich angeordneten Durchgangskanal 11 zwei Temperierkanäle 12, 13. Die Temperierkanäle 12, 13 enden vor einer Verjüngung 36, die den Übergang vom distalen Katheterabschnitt 16 zur Katheterspitze 18 bildet. Die Verjüngung verjüngt sich zur Katheterspitze 18 und bildet im Wesentlichen eine kegelstumpfförmige Außenkontur.

Fig. 17b zeigt einen Querschnitt durch den Katheterschlauch 10 im distalen Katheterabschnitt 16. Gut erkennbar sind die lungenflügelartigen Temperierkanäle 12, 13 und der dezentrale Durchgangskanal 11, der einen kreisrunden Querschnitt aufweist. In Fig. 17c ist die Querschnittsgeometrie des Katheterschlauchs 10 im Bereich der Verjüngung 36 gezeigt. Dort hat die Außenwand 20 des Katheterschlauchs 10 eine unregelmäßige Wandstärke. Es ist auch möglich, dass sich die Temperierkanäle 12, 13 in die Verjüngung 36 fortsetzen und sich ebenfalls (graduell) verjüngen, d.h. die Temperierkanäle 12, 13 können im Bereich der Verjüngung36 einen distal sich reduzierenden Querschnittsdurchmesser aufweisen. Insofern können die Temperierkanäle 12, 13 in bestimmten Querschnittsansichten der Verjüngung 36 erkennbar sein. Vorzugsweise ist die Wandstärke im Bereich der Verlängerung der im proximalen Katheterabschnitt 15 angeordneten Temperierkanäle 12, 13 größer als auf einer bezüglich des Durchgangskanals 11 diametral gegenüberliegenden Seite.

Fig. 17d zeigt einen Querschnitt durch den innerhalb des Durchgangskanals 11 angeordneten Dilatator 35, insbesondere im Bereich einer Dilatatorspitze 35a. Der Dilatator weist einen Führungskanal 35b auf, der im Wesentlichen über die gesamte Länge des Dilatators 35 einen konstanten Querschnittsdurchmesser aufweist. Durch den Führungskanal 35b kann der Dilatator über einen Führungsdraht an den Behandlungsort geführt werden.

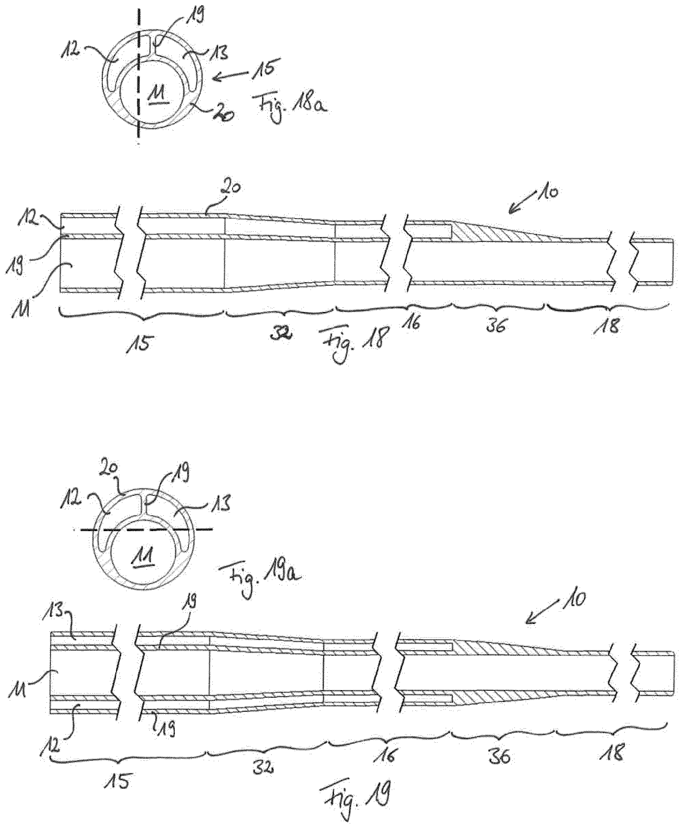

In den Figuren 18, 18a, 19 und 19b sind weitere Schnitte durch den Katheter bzw. Katheterschlauch 10 gemäß Fig. 17 gezeigt, wobei auch ein proximaler Katheterabschnitt 15 dargestellt ist. Es ist erkennbar, dass der Katheterschlauch 10 einen Übergangsabschnitt 32 aufweist, der zwischen dem proximalen Katheterabschnitt 15 und dem distalen Katheterabschnitt 16 angeordnet ist. An den distalen Katheterabschnitt 16 schließt sich über die Verjüngung 36 die Katheterspitze 18 an. Die Temperierkanäle 12, 13 des Katheterschlauchs 10 erstrecken sich über den Übergangsabschnitt 32 in den distalen Katheterabschnitt 16, der die (hier nicht dargestellten) Wärmetauscherelemente 14 trägt. Die Wandstärke der Innenwand 19 und der Außenwand 20 kann im Übergangsabschnitt 32 konstant sein. Vorzugsweise ändert sich jedoch die Wandstärke direkt proportional zur Änderung des Außendurchmessers bzw. zur Änderung des Querschnittsdurchmessers der Temperierkanäle 12, 13.

Die Katheterspitze 18 kann im Allgemeinen ein Material aufweisen, das weicher bzw. flexibler als das Material des proximalen und/oder distalen Katheterabschnitts 15, 16 ist. Damit wird erreicht, dass sich die Katheterspitze 18 im Bereich von Gefäßverzweigungen, insbesondere Bifurkationen, gut in das gewünschte Zielgefäß führen lässt. Außerdem wird auf diese Weise ein guter Kontakt mit einem Dilatator 36 sichergestellt, was insbesondere bei der Schleusenvariante Vorteile mit sich bringt. Schließlich hat die Katheterspitze 18 Vorteile bei der Aspiration von Thromben, da sich durch die Katheterspitze 18 die distale Öffnung des Durchgangskanals 11 weiter distal befindet. Insofern hat es sich als zweckmäßig erwiesen, wenn die Katheterspitze 18 eine Länge aufweist, die wenigstens 0,5 cm, insbesondere wenigstens 1 cm, insbesondere wenigstens 2 cm, insbesondere wenigstens 3 cm, insbesondere wenigstens 4 cm, insbesondere wenigstens 5 cm, beträgt. Höchstens sollte die Katheterspitze 18 eine Länge von 8 cm aufweisen. Generell ist außerdem vorgesehen, dass die Katheterspitze 18 nur einen einzigen Kanal, insbesondere den Durchgangskanal 11, beinhaltet. Vorzugsweise weist die Katheterspitze 18 eine sich nach distal verjüngende Außenkontur auf.

In diesem Zusammenhang wird darauf hingewiesen, dass sich die Darstellung gemäß Fig. 17 bis 19 sowohl auf die Schleusenvariante, als auch die Kathetervariante des Katheters beziehen kann. Bei der Schleusenvariante kann der Katheter, insbesondere der Katheterschlauch 10, selbst die Funktion einer Schleuse übernehmen, so dass eine zusätzliche Schleuse zur Punktierung eines Blutgefäßes und zur Führung des Katheters nicht notwendig ist. Bei der Kathetervariante wird der Katheterschlauch 10 über eine zuvor in das Blutgefäß eingeführte Schleuse an den Behandlungsort geführt.

Hinsichtlich der Gestaltung des Katheterschlauchs 10 an seinem proximalen Ende ist vorgesehen, einen Luer-Lock-Adapter zu verwenden. Der Luer-Lock-Adapter ist vorzugsweise fest mit dem Katheterschlauch 10 verbunden und ermöglicht einen Zugang, insbesondere für Führungsdrähte und/oder Fluide, zu den verschiedenen Kanälen des Katheterschlauchs 10. Insbesondere ist der Luer-Lock-Adapter derart ausgebildet, dass über diesen ein Zugang zu den Temperierkanälen 12, 13 sowie zum Durchgangskanal 11 möglich ist.

Hinsichtlich der Dimensionen eines Katheterschlauchs eines erfindungsgemäßen Katheters (Kathetervariante), der insbesondere über eine Schleuse der Größe 8 French zuführbar ist, sind folgende Kennwerte bevorzugt:

| | Proximaler Katheterabschnitt | Distaler Katheterabschnitt |
|---|---|---|
| Außendurchmesser [mm] | 2,3 - 2,8 | 1,9 - 2,3 |
| | vorteilhaft: 2,5 - 2,7 | vorteilhaft: 2,0 - 2,2 |
| | bevorzugt: | bevorzugt: |
| Gesamtlänge [m] | 0,9 - 1,3 | |
| | vorteilhaft: 1,0-1,2 | |
| | bevorzugt: 1,1 | |
| Länge des distalen Katheterabschnitts bzw. Ballonbereichs [mm] | | 40 - 160 |
| | | vorteilhaft: 60 - 140 |
| | | vorteilhaft: 80 - 120 |
| | | vorteilhaft: 90 - 110 |
| | | bevorzugt: 100 |
| Länge des Übergangsbereichs [mm] | mindestens 10 | |
| | und höchstens 120 | |
| | oder höchstens 100 | |
| | oder höchstens 80 | |
| | oder höchstens 60 | |
| | oder höchstens 40 | |
| | oder höchstens 30 | |
| | oder höchstens 20 | |
| Wandstärke [µm] | 100 - 300 | 50 - 250 |
| | vorteilhaft: 150 - 250 | vorteilhaft: 100 - 200 |
| | bevorzugt: 200 | bevorzugt: 150 |
| Verhältnis Außendurchmesser distaler Katheterabschnitt / Außendurchmesser proximaler Katheterabschnitt | 0,6 - 0,95 | |
| | vorteilhaft: 0,7 - 0,9 | |
| | bevorzugt: 0,8 | |
| Differenz zwischen Außendurchmesser distaler Katheterabschnitt und Außendurchmesser proximaler Katheterabschnitt | 200 - 1000 | |
| | vorteilhaft: 300 - 800 | |
| | vorteilhaft: 400 - 600 | |
| | bevorzugt: 500 | |

Hinsichtlich der Dimensionen eines Katheterschlauchs 10 eines erfindungsgemäßen Katheter (Schleusenvariante), der im Rahmen einer Doppelfunktion sowohl als Schleuse für einen 8 French Katheter, als auch als Katheter wirkt, sind folgende Kennwerte bevorzugt:

| | Proximaler Schleusenabschnitt | Distaler Schleusenabschnitt |
|---|---|---|
| Außendurchmesser [mm] | | 2,3 - 3,6 |
| | 3,2 - 4,0 | vorteilhaft: 2,6 - 3,4 |
| | vorteilhaft: 3,4 - 3,9 | vorteilhaft: 2,8 - 3,3 |
| | vorteilhaft: 3,5 - 3,8 | vorteilhaft: 2,9 - 3,2 |
| | bevorzugt: 3,6 - 3,7 | bevorzugt: 3,0-3,1 |
| Innendurchmesser des Durchgangskanals [mm] | | |
| - 5F Variante | | 1,7 - 2,1 |
| | | Bevorzugt: 1,8 - 2,0 |
| - 6F Variante | | 2,1 - 2,4 |
| | | Bevorzugt: 2,2 - 2,3 |
| Gesamtlänge [m] | 0,8 - 1,0 | |
| | bevorzugt: 0,9 | |
| Länge [mm] | | 40 - 120 |
| | | vorteilhaft: 60 - 100 |
| | | vorteilhaft: 70 - 90 |
| | | bevorzugt 80 |
| Länge des Übergangsbereichs [mm] | mindestens 10 | |
| | und höchstens 120 | |
| | oder höchstens 100 | |
| | oder höchstens 80 | |
| | oder höchstens 60 | |
| | oder höchstens 40 | |
| | oder höchstens 30 | |
| | oder höchstens 20 | |
| Wandstärke [µm] | 100 - 400 | 50 - 300 |
| | vorteilhaft: 200 - 300 | vorteilhaft: 150-250 |
| | bevorzugt: 250 | bevorzugt: 200 |
| Verhältnis Außendurchmesser distaler Katheterabschnitt / Außendurchmesser proximaler Katheterabschnitt | 0,6 - 0,95 | |
| | vorteilhaft: 0,7 - 0,9 | |
| | bevorzugt 0,8 | |
| Differenz zwischen Außendurchmesser distaler Katheterabschnitt und Außendurchmesser proximaler Katheterabschnitt | 100 - 1000 | |
| | vorteilhaft: 200 - 1000 | |
| | vorteilhaft: 300 - 800 | |
| | vorteilhaft: 400 - 600 | |
| | bevorzugt: 500 | |

Wie aus den Tabellen hervorgeht, wir im Rahmen der vorliegenden Anmeldung bei den Größenangaben zwischen einer Schleuse und einem Katheter unterschieden. Bei einem Katheter bezieht sich die Größenangabe in French auf den Außendurchmesser des Katheterschlauchs. Eine Schleuse mit derselben Größenangabe weist hingegen einen größeren Außendurchmesser auf, da mit die Größenangabe bei einer Schleuse vielmehr auf die maximale Größe eines Katheters hinweist, der über die Schleuse zugeführt werden kann. Der Außendurchmesser einer 8 French Schleuse entspricht daher vielmehr dem Außendurchmesser eines 8 French Katheters zuzüglich der Wandstärke der Schleuse.

Grundsätzlich ist für die vorliegende Erfindung eine Größe von 8 French für den Katheterschlauch bevorzugt, wobei sich diese Größenangabe bei der Kathetervariante auf den Außendurchmesser und bei de Schleusenvariante auf den Innendurchmesser des Durchgangskanals (zzgl. einer Toleranz, damit ein 8 French-Katheter durch den Durchgangskanal 11 geschoben werden kann) bezieht. Es sind jedoch auch andere Größen möglich. Der Katheterschlauch 10 kann auch skaliert werden, so dass Größen von 4, 6, 5 oder 7 French bis 9, 10, 11 oder 12 French erreicht werden. Dabei bleiben die Dimensionen der einzelnen Kanäle sowie Innen- und Außenwände des Katheterschlauchs 10 vorzugweise zumindest annähernd proportional zueinander.

### Bezugszeichenliste

- 10: Katheterschlauch
- 11: Durchgangskanal
- 12: Erster Temperierkanal
- 13: Zweiter Temperierkanal
- 14: Wärmetauscherelement
- 15: Proximaler Katheterabschnitt
- 16: Distaler Katheterabschnitt
- 17: Aufnahmeraum
- 18: Katheterspitze
- 19: Innenwand
- 20: Außenwand
- 21: Innere Schicht
- 22: Äußere Schicht
- 23: Innenschlauch
- 24: Außenschlauch
- 25: Abstandshalter
- 26: Isolierraum
- 27: Durchgangsöffnung
- 28: Proximale Außenumfangsebene
- 29: Distale Außenumfangsebene
- 30: Proximaler Abschlussflansch
- 31: Distaler Abschlussflansch
- 32: Übergangsabschnitt
- 33: Distale Fluidöffnung
- 34: Proximale Fluidöffnung
- 35: Dilatator
- 35a: Dilatatorspitze
- 35b: Führungskanal
- 36: Verjüngung

## Patentansprüche

1. Medizinischer Katheter zur hypothermischen Behandlung mit einem Katheterschlauch (10), der wenigstens einen Durchgangskanal (11) und wenigstens zwei Temperierkanäle (12, 13) aufweist, und mit wenigstens einem Wärmetauscherelement (14), insbesondere einem expandierbaren Ballon, das in einem distalen Katheterabschnitt (15) des Katheterschlauchs (10) angeordnet und mit den Temperierkanälen (12, 13) fluidverbunden ist derart, dass ein Temperierkreislauf gebildet ist,
wobei
der Katheterschlauch (10) im distalen Katheterabschnitt (16) einen kleineren Außendurchmesser als in einem proximalen Katheterabschnitt (15) aufweist.

2. Katheter nach Anspruch 1,
**dadurch gekennzeichnet, dass**
der distale Katheterabschnitt (16) eine höhere Biegeflexibilität als der proximale Katheterabschnitt (15) aufweist.

3. Katheter nach Anspruch 1 oder 2,
**dadurch gekennzeichnet, dass**
der Katheterschlauch (10) im distalen Katheterabschnitt (16) eine kleinere Wandstärke als im proximalen Katheterabschnitt (15) aufweist.

4. Katheter nach einem der vorhergehenden Ansprüche,
**dadurch gekennzeichnet, dass**
der Katheterschlauch (10) ein konstantes Querschnittsprofil, insbesondere einen konstanten Innendurchmesser, aufweist.

5. Katheter nach einem der vorhergehenden Ansprüche,
**dadurch gekennzeichnet, dass**
das Wärmetauscherelement (14) komprimierbar ist derart, dass das Wärmetauscherelement (14) im komprimierten Zustand einen Querschnittsdurchmesser aufweist, der höchstens dem Außendurchmesser des proximalen Katheterabschnitts (15) entspricht.

6. Katheter nach einem der vorhergehenden Ansprüche,
**dadurch gekennzeichnet, dass**
am distalen Katheterabschnitt (16) zwischen einer Außenumfangsebene (29) des distalen Katheterabschnitts (16) und einer Außenumfangsebene (28) des proximalen Katheterabschnitts (15) ein Aufnahmeraum (17) für das Wärmetauscherelement (14) gebildet ist, wobei das Wärmetauscherelement (14) komprimierbar ist derart, dass das Wärmetauscherelement (14) vollständig innerhalb des Aufnahmeraums (17) anordenbar ist.

7. Katheter nach einem der vorhergehenden Ansprüche,
**dadurch gekennzeichnet, dass**
der Katheterschlauch (10) einstückig ausgebildet ist.

8. Katheter nach einem der vorhergehenden Ansprüche,
**dadurch gekennzeichnet, dass**
der Katheterschlauch (10) zumindest im proximalen Katheterabschnitt (15) eine mehrschichtige, insbesondere zweischichtige, Außenwand (20) aufweist.

9. Katheter nach Anspruch 8,
**dadurch gekennzeichnet, dass**
die Außenwand (20) im proximalen Katheterabschnitt (15) durch eine innere Schicht (21) und eine äußere Schicht (22) und/oder im distalen Katheterabschnitt (16) durch die innere Schicht (21) gebildet ist.

10. Katheter nach Anspruch 8 oder 9,
**dadurch gekennzeichnet, dass**
zumindest die äußere Schicht (22) eine Wärmeleitfähigkeit aufweist, die höchstens 0,2 Wm⁻¹K⁻¹, insbesondere höchstens 0,15 Wm⁻¹K⁻¹, insbesondere höchstens 0,1 Wm⁻¹K⁻¹, insbesondere höchstens 0,08 Wm⁻¹K⁻¹, beträgt.

11. Katheter nach einem der vorhergehenden Ansprüche,
**dadurch gekennzeichnet, dass**
zumindest die Temperierkanäle (12, 13) im proximalen Katheterabschnitt (15) eine größere Querschnittsfläche als im distalen Katheterabschnitt (16) aufweisen.

12. Katheter nach einem der vorhergehenden Ansprüche,
**dadurch gekennzeichnet, dass**
die Querschnittsgeometrie des distalen Katheterabschnitt (16) durch eine Skalierung der Querschnittsgeometrie des proximalen Katheterabschnitts (15) gebildet ist.

13. Katheter nach einem der vorhergehenden Ansprüche,
**dadurch gekennzeichnet, dass**
der Katheterschlauch (10) eine Katheterspitze (18) aufweist, durch die sich nur der Durchgangskanal (11) erstreckt.

14. Behandlungssystem mit einem Katheter nach einem der vorhergehenden Ansprüche und einer selbstexpandierbaren Vorrichtung, insbesondere einem Rekanalisationselement, die mit einem länglichen Führungselement, insbesondere ein Transportdraht, verbunden und mit dem Führungselement, insbesondere dem Transportdraht, längsverschiebbar im Durchgangskanal (11) angeordnet ist.

15. Verfahren zur Herstellung eines Katheters und/oder eines Behandlungssystems nach einem der vorhergehenden Ansprüche, wobei der Katheterschlauch (10) aus einem einzigen Material oder aus mehreren unterschiedlichen Materialien durch einen Extrusionsprozess einstückig ausgebildet wird.

## Claims

1. A medical catheter for hypothermic treatment, comprising a catheter tube (10), which has at least one through-duct (11) and at least two temperature-control ducts (12, 13), and comprising at least one heat exchanger element (14), in particular an expandable balloon, which is arranged in a distal catheter portion (15) of the catheter tube (10) and is fluidically connected to the temperature-control ducts (12, 13) in such a way that a temperature-control circuit is formed,
wherein the catheter tube (10) in the distal catheter portion (16) has a smaller outer diameter than in a proximal catheter portion (15).

2. The catheter according to claim 1,
**characterised in that**
the distal catheter portion (16) has a higher bending flexibility than the proximal catheter portion (15).

3. The catheter according to claim 1 or 2,
**characterised in that**
the catheter tube (10) in the distal catheter portion (16) has a smaller wall thickness than in the proximal catheter portion (15).

4. The catheter according to any one of the preceding claims,
**characterised in that**
the catheter tube (10) has a constant cross-sectional profile, in particular a constant inner diameter.

5. The catheter according to any one of the preceding claims,
**characterised in that**
the heat exchanger element (14) is compressible, in such a way that the heat exchanger element (14) in the compressed state has a cross-sectional diameter that at most corresponds to the outer diameter of the proximal catheter portion (15).

6. The catheter according to any one of the preceding claims,
**characterised in that**
a receiving space (17) for the heat exchanger element (14) is formed on the distal catheter portion (16) between an outer peripheral level (29) of the distal catheter portion (16) and an outer peripheral level (28) of the proximal catheter portion (15), wherein the heat exchanger element (14) is compressible in such a way that the heat exchanger element (14) can be arranged fully within the receiving space (17).

7. The catheter according to any one of the preceding claims,
**characterised in that**
the catheter tube (10) is formed in one piece.

8. The catheter according to any one of the preceding claims,
**characterised in that**
the catheter tube (10) has a multi-layer, in particular two-layer, outer wall (20) at least in the proximal catheter portion (15).

9. The catheter according to claim 8,
**characterised in that**
the outer wall (20) is formed in the proximal catheter portion (15) by an inner layer (21) and an outer layer (22) and/or in the distal catheter portion (16) by the inner layer (21).

10. The catheter according to claim 8 or 9,
**characterised in that**
at least the outer layer (22) has a thermal conductivity that is at most 0.2 Wm⁻¹K⁻¹, in particular at most 0.15 Wm⁻¹K⁻¹, in particular at most 0.1 Wm⁻¹K⁻¹, in particular at most 0.08 Wm⁻¹K⁻¹.

11. The catheter according to any one of the preceding claims,
**characterised in that**
at least the temperature-control ducts (12, 13) in the proximal catheter portion (15) have a larger cross-sectional area than in the distal catheter portion (16) .

12. The catheter according to any one of the preceding claims,
**characterised in that**
the cross-sectional geometry of the distal catheter portion (16) is formed by a scaling of the cross-sectional geometry of the proximal catheter portion (15).

13. The catheter according to any one of the preceding claims,
**characterised in that**
the catheter tube (10) has a catheter tip (18), through which only the through-duct (11) extends.

14. A treatment system comprising a catheter according to any one of the preceding claims and a self-expandable device, in particular a rechannelling element, which is connected to an elongated guide element, in particular a transport wire, and arranged with the guide element, in particular the transport wire, in a longitudinally displaceable manner in the through-duct (11).

15. A method for producing a catheter and/or a treatment system according to any one of the preceding claims, wherein the catheter tube (10) is formed in one piece from a single material or from a plurality of different materials by an extrusion process.

## Revendications

1. Cathéter médical, destiné au traitement hypothermique, pourvu d'un flexible de cathéter (10), qui comporte au moins un conduit de passage (11) et au moins deux conduits régulateurs thermiques (12, 13) et pourvu d'au moins un élément échangeur thermique (14), en particulier un ballon expansible qui est placé dans un tronçon distal de cathéter (15) du flexible de cathéter (10) et qui est relié fluidiquement avec les conduits régulateurs thermiques (12, 13), de sorte à créer un circuit de régulation thermique, dans le tronçon distal du cathéter (16), le flexible de cathéter (10) présentant un diamètre extérieur inférieur à celui dans un tronçon proximal du cathéter (15).

2. Cathéter selon la revendication 1, **caractérisé en ce que** le tronçon distal du cathéter (16) fait preuve d'une souplesse en flexion supérieure à celle du tronçon proximal du cathéter (15).

3. Cathéter selon la revendication 1 ou 2, **caractérisé en ce que** dans le tronçon distal du cathéter (16), le flexible de cathéter (10) présente une épaisseur de paroi inférieure à celle du tronçon proximal du cathéter (15).

4. Cathéter selon l'une quelconque des revendications précédentes, **caractérisé en ce que** le flexible de cathéter (10) présente un profil de section transversale constant, en particulier un diamètre intérieur constant.

5. Cathéter selon l'une quelconque des revendications précédentes, **caractérisé en ce que** l'élément échangeur thermique (14) est compressible, de telle sorte qu'à l'état comprimé, l'élément échangeur thermique, (14) présente un diamètre de section transversale qui correspond au maximum au diamètre extérieur du tronçon proximal du cathéter (15).

6. Cathéter selon l'une quelconque des revendications précédentes, **caractérisé en ce que** sur le tronçon distal du cathéter (16), entre un plan périphérique extérieur (29) du tronçon distal du cathéter (16) et un plan périphérique extérieur (28) du tronçon proximal du cathéter (15) est formé un espace de logement (17) pour l'élément échangeur thermique (14), l'élément échangeur thermique (14) étant compressible, de telle sorte que l'élément échangeur thermique puisse être placé intégralement à l'intérieur de l'espace de logement (17).

7. Cathéter selon l'une quelconque des revendications précédentes, **caractérisé en ce que** le flexible de cathéter (10) est conçu en monobloc.

8. Cathéter selon l'une quelconque des revendications précédentes, **caractérisé en ce qu'**au moins dans le tronçon proximal du cathéter (15), le flexible de cathéter (10) comporte une paroi extérieure (20) à plusieurs couches, en particulier à deux couches.

9. Cathéter selon la revendication 8, **caractérisé en ce que** dans le tronçon proximal du cathéter (15), la paroi extérieure (20) est formée d'une couche intérieure (21) et d'une couche extérieure (22) et/ou dans le tronçon distal du cathéter (16), est formée par la couche intérieure (21).

10. Cathéter selon la revendication 8 ou 9, **caractérisé en ce qu'**au moins la couche extérieure (22) fait preuve d'une conductibilité thermique, qui est d'un maximum de 0,2 Wm⁻¹K⁻¹, en particulier d'un maximum de 0,15 Wm⁻¹K⁻¹, en particulier d'un maximum de 0,1 Wm⁻¹K⁻¹, en particulier d'un maximum de 0,08 Wm⁻¹K⁻¹.

11. Cathéter selon l'une quelconque des revendications précédentes, **caractérisé en ce que** dans le tronçon proximal du cathéter (15), au moins les conduits régulateurs thermiques (12, 13) présentent une surface de section transversale supérieure à celle dans le tronçon distal du cathéter (16).

12. Cathéter selon l'une quelconque des revendications précédentes, **caractérisé en ce que** la géométrie de section transversale du tronçon distal du cathéter (16) est formée par une graduation de la géométrie de section transversale du tronçon proximal du cathéter (15).

13. Cathéter selon l'une quelconque des revendications précédentes, **caractérisé en ce que** le flexible de cathéter (10) comporte une pointe de cathéter (18) à travers laquelle s'étend uniquement le conduit de passage (11).

14. Système de traitement, pourvu d'un cathéter selon l'une quelconque des revendications précédentes et d'un dispositif auto-expansible, en particulier d'un élément de recanalisation, qui est relié avec un élément de guidage allongé, en particulier un fil métallique de transport, et qui par l'élément de guidage, en particulier le fil métallique de transport, est placé de manière déplaçable en longueur dans le conduit de passage (11).

15. Procédé, destiné à la fabrication d'un cathéter et/ou d'un système de traitement selon l'une quelconque des revendications précédentes, le flexible de cathéter (10) étant conçu en monobloc, en une matière unique ou en plusieurs matières différentes par un procédé d'extrusion.
